# EUROPÄISCHE PATENTSCHRIFT

(11) **EP 1 038 941 B1**
(45) Veröffentlichungstag und Bekanntmachung des Hinweises auf die Patenterteilung: **21.05.2003**
(21) Anmeldenummer: 00103769.6
(22) Anmeldetag: 23.02.2000
(51) Int. Cl.: C09K 19/58, C09K 19/04, C09K 19/20, C09K 19/00, A61K 7/00, A61K 7/42

(54) **Chirale Verbindungen und deren Verwendung als chirale Dotierstoffe zur Herstellung von cholesterische-fl-ssigkristallinen Zusammensetzungen**
Chiral compounds and their use as chiral dopants for preparing cholesteric liquid crystal compositions
Composés chiraux et leur utilisation comme dopants chiraux pour la préparation de compositions liquides cristallines cholestériques

(30) Priorität: 25.03.1999 DE 19913604
(43) Veröffentlichungstag der Anmeldung: 27.09.2000
(73) Patentinhaber: BASF AKTIENGESELLSCHAFT, 67056 Ludwigshafen (DE)
(72) Erfinder: Meyer, Frank, Dr., 69115 Heidelberg (DE); Schuhmacher, Peter, Dr., 68163 Mannheim (DE); Prechtl, Frank, Dr., 60318 Frankfurt (DE)

(56) Entgegenhaltungen:
- EP-A- 0 962 222
- EP-A- 0 998 900
- WO-A-97/00600
- DE-A- 4 342 280
- DE-A- 19 738 369

## Beschreibung

Die Erfindung betrifft chirale Verbindungen und deren Verwendung als chirale Dotierstoffe zur Herstellung von cholesterisch-flüssigkristallinen Zusammensetzungen, die als photostabile UV-Filter in kosmetischen und pharmazeutischen Zubereitungen zum Schutz der menschlichen Epidermis oder menschlichen Haare gegen UV-Strahlung, speziell im Bereich von 280 bis 450 nm, eingesetzt werden können.

Cholesterische Flüssigkristalle (Cholesteric Liquid Crystal; CLC) reflektieren zirkular polarisierte elektromagnetische Strahlung in einem von der helikalen Struktur des CLC abhängigen Wellenbereich. Die zentrale Wellenlänge des Reflexionsbandes wird durch die Ganghöhe p der helikalen Struktur bestimmt, die Breite des Bandes durch die optische Anisotropie der Mesogene. Die zentrale Wellenlänge des Reflexionsbandes, die im folgenden als Reflexionswellenlänge bezeichnet wird, ist vom Betrachtungswinkel abhängig. Die Drehrichtung des reflektierten Lichts entspricht dem Drehsinn der cholesterischen Helix.

Cholesterische Flüssigkristallmischungen enthalten in der Regel eine oder mehrere optisch aktive Komponenten zur Induktion einer chiralen Struktur. Beispielsweise können cholesterische Flüssigkristallmischungen aus einem nematischen Basismaterial und einem oder mehreren optisch aktiven Dotierstoffen bestehen. Dabei erzeugen diese im Nematen entweder eine rechts- oder linkshändige Verdrillung, die die Drehrichtung des reflektierten zirkularpolarisierten Lichts bestimmt.

Als chirale Dotierstoffe für flüssigkristalline Phasen sind zahlreiche Verbindungen bekannt (z.B. aus DE-A 43 42 280, DE-A 195 41 820 und DE-A 196 11 101 sowie aus GB-A-2 314 839 und WO 98/00428).

**Weitere Beispiele für chirale Dotierstoffe sowie daraus hergestellte Pigmente finden sich in DE-A 197 38 368, EP-A-0 962 222, DE-A 43 42 280 und WO 97/00600.**

Für linkshelikale Materialien kommen häufig Cholesterinverbindungen in Frage, die außer der Chiralität ausreichend mesogene Eigenschaften mitbringen, um eine stabile Mesophase zu erzeugen.

Solche Verbindungen sind beispielsweise beschrieben von H. Finkelmann, H. Ringsdorf et al., in Makromol. Chem. 179, 829-832 (1978). Diese Verbindungen haben allerdings den Nachteil einer komplizierten Synthese und eines hohen Herstellungspreises.

Es bestand nun die Aufgabe, neue chirale Verbindungen bereitzustellen, die für die Herstellung von cholesterisch-flüssigkristallinen Zusammensetzungen geeignet sind und die die oben genannten Nachteile nicht aufweisen.

Diese Aufgabe wurde erfindungsgemäß gelöst durch chirale Dotierstoffe der allgemeinen Formel I,

Z¹-Y¹-(A¹)ₘ-Y²-M-Y³-X-Z² I

in der die Variablen unabhängig voneinander folgende Bedeutung haben:
- A¹: ein Spacer, ausgewählt aus der Gruppe, bestehend aus
-(CH₂)_{q}-, -(CH₂CH₂O)ᵣCH₂CH₂-, -CH₂CH₂SCH₂CH₂-, -CH₂CH₂NHCH₂CH₂-, 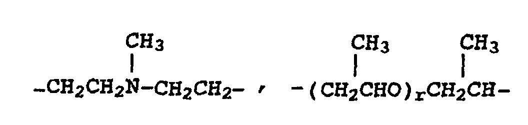 und 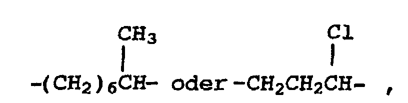 wobei r für 1 bis 3 und q für 1 bis 12 steht.
- Y¹ bis Y³: eine chemische Bindung, -O-, -S-, -C(=O)-, -C(=O)-O-, -O-C(=O)-, -CH=CH-C(=O)-O-, -O-C(=O)-O-, -C(=O)-N(R)- oder -(R)N-C(=O)-, -CH₂-O-, -O-CH₂-, -CH=N-, -N=CH- oder -N=N-;
- M: eine mesogene Gruppe, ausgewählt aus der Gruppe, bestehend aus 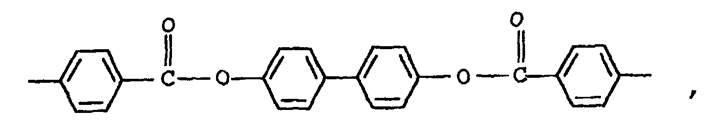 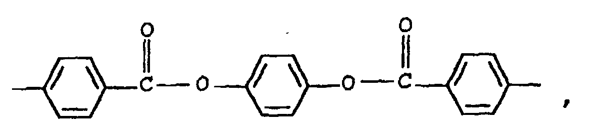 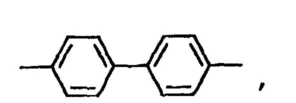 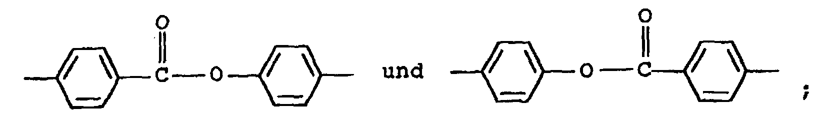
- R: Wasserstoff, C₁-C₄-Alkyl;
- Z¹ und Z²: Wasserstoff, C₁-C₄-Alkyl, eine polymerisierbare Gruppe oder ein Rest, der eine polymerisierbare Gruppe trägt, ausgewählt aus
CH₂=CH- , CH≡C- , 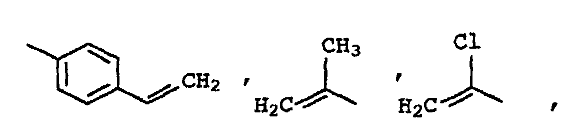 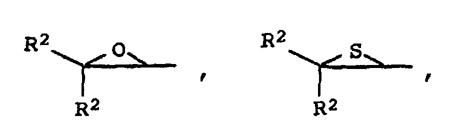 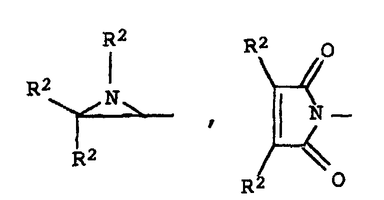 -N=C=O, -N=C=S, -O-C≡N, -COOH, -OH und NH₂,
wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten;
- X: ein Dianhydrohexitrest, ausgewählt aus der Gruppe, bestehend aus Dianhydrosorbit, Dianhydromannit und Dianhydroidit;
- m: 0 oder 1,
wobei die Reste Z¹, Z², Y¹ bis Y³ gleich oder verschieden sein können und mindestens ein Rest Z¹ oder Z² eine polymerisierbare Gruppe oder einen Rest, der eine polymerisierbare Gruppe enthält, bedeutet.

Bevorzugte Spacer sind Ethylen, Propylen, n-Butylen, n-Pentylen und n-Hexylen.

Ferner ist es auch möglich, den mesogenen Rest M ohne Spacer direkt mit dem Rest Z¹ zu verknüpfen. In diesen Fall stehen m für 0 sowie Y¹ oder Y² für eine chemische Bindung.

Als Alkylreste seien für R sowie für Z¹ und Z² bevorzugt Methyl, Ethyl, n-Propyl, 1-Methylethyl, n-Butyl, 1-Methylpropyl-, 2-Methylpropyl oder 1,1-Dimethylethyl genannt.

Bevorzugte Reste für Z¹ und Z² sind: 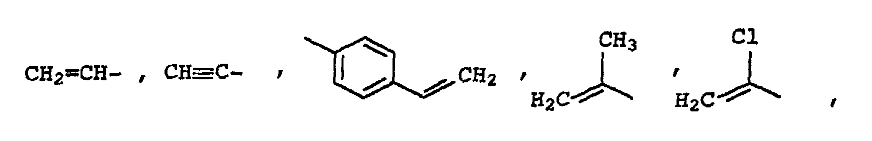 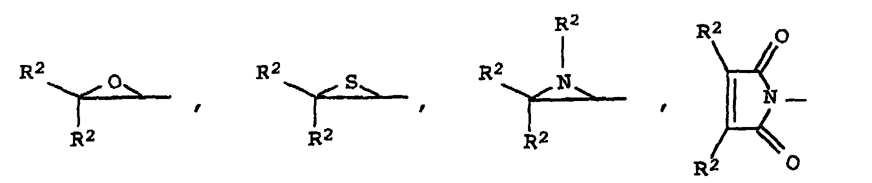 -N=C=O, -N=C=S, -O-C≡N, -COOH, -OH oder NH₂
wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten. Von den reaktiven polymerisierbaren Gruppen können die Cyanate spontan zu Cyanuraten trimerisieren und sind daher bevorzugt. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen wie Styrol. Die komplementären reaktiven Gruppen können dabei entweder in einer zweiten erfindungsgemäßen Verbindung vorhanden sein, die mit der ersteren gemischt wird, oder sie können durch Hilfsverbindungen, die 2 oder mehr dieser komplementären Gruppen enthalten, in das polymere Netzwerk eingebaut werden.

Besonders bevorzugte Gruppierungen Z¹ bzw. Z² sind Acrylat und Methacrylat.

Y¹-Y³ können die oben genannten Bedeutungen haben, wobei unter einer chemischen Bindung eine kovalente Einfachbindung verstanden werden soll.

Als Dianhydrohexitrest kommen Reste, ausgewählt aus der Gruppe, bestehend aus Dianhydrosorbit, Dianhydromannit und Dianhydroidit in Frage. Bevorzugt Reste sind Dianhydrosorbit und Dianhydromannit, besonders bevorzugt Dianhydromannit.

Besonders bevorzugt sind die Dianhydromannit-enthaltenden chiralen Dotierstoffe der allgemeinen Formel Ia, 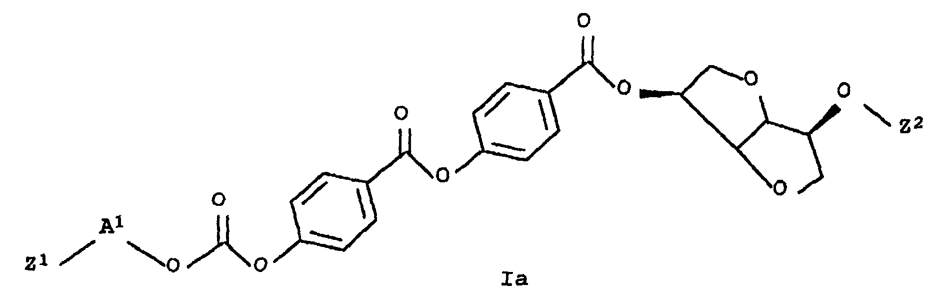 in der die Variablen unabhängig voneinander folgende Bedeutung haben:
- A¹: ein Spacer, ausgewählt aus der Gruppe, bestehend aus Ethylen, Propylen, n-Butylen, n-Pentylen und n-Hexylen;
- Z¹ und Z²: CH₂=CH-,

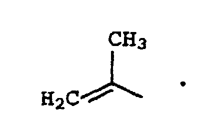

Die Herstellung der oben genannten Verbindungen erfolgt in an sich bekannter Weise gemäß den in DE-A-195 32 408, DE-A-44 08 171, EP-A-0 750 029 sowie in WO 95/16007 beschriebenen Verfahren. Bezüglich näherer Einzelheiten sei auf diese Schriften verwiesen.

Die erfindungsgemäßen chiralen Verbindungen der Formel I eignen sich u.a. als chirale Dotierstoffe zur Herstellung von cholesterisch-flüssigkristallinen Zusammensetzungen, insbesondere von solchen cholesterischen Flüssigkristallen mit linkshändiger Verdrillung.

Gegenstand der Erfindung sind daher auch cholesterisch-flüssigkristalline Zusammensetzungen, enthaltend
a) mindestens ein chirales flüssigkristallines polymerisierbares Monomeres der allgemeinen Formel I,

   Z¹-Y¹-(A¹)ₘ-Y²-M-Y³-X-Z² I

   mit dem eine cholesterisch-flüssigkristalline Phase mit einer Ganghöhe von kleiner 450 nm erzielt werden kann, in der die Variablen unabhängig voneinander folgende Bedeutung haben:
   - A¹: ein Spacer, ausgewählt aus der Gruppe, bestehend aus
   -(CH₂)_{q}-, -(CH₂CH₂O)ᵣCH₂CH₂-, -CH₂CH₂SCH₂CH₂-, -CH₂CH₂NHCH₂CH₂-, 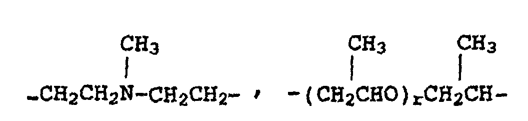 und 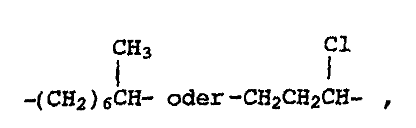 wobei r für 1 bis 3 und q für 1 bis 12 steht.
   - Y¹ bis Y³: eine chemische Bindung, -O-, -S-, -C(=O)-, -C(=O)-O-, -O-C(=O)-, -CH=CH-C(=O)-O-, -O-C(=O)-O-, -C(=O)-N(R)-oder -(R)N-C(=O)-, -CH₂-O-, -O-CH₂-, -CH=N-, -N=CH- oder -N=N-;
   - M: eine mesogene Gruppe, ausgewählt aus der Gruppe, bestehend aus 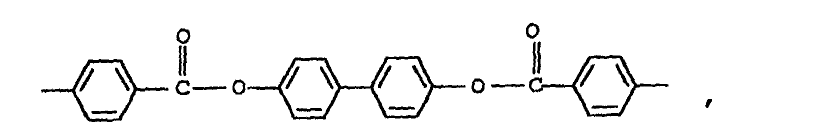 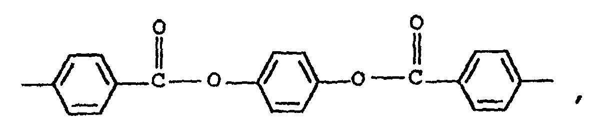 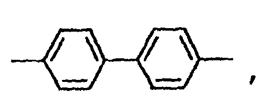 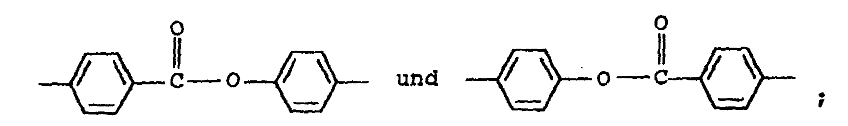
   - R: Wasserstoff, C₁-C₄-Alkyl;
   - Z¹ und Z²: Wasserstoff, C₁-C₄-Alkyl, eine polymerisierbare Gruppe oder ein Rest, der eine polymerisierbare Gruppe trägt, ausgewählt aus
   CH₂=CH- , CH≡C- , 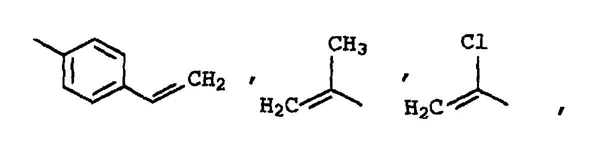 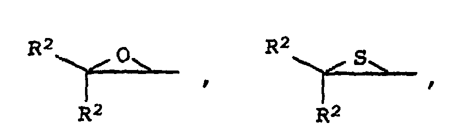 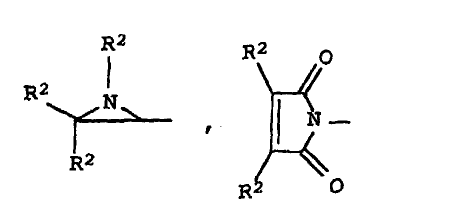 -N=C=O, -N=C=S, -O-C≡N, -COOH, -OH und NH₂,
   wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten;
   - X: ein Dianhydrohexitrest, ausgewählt aus der Gruppe, bestehend aus Dianhydrosorbit, Dianhydromannit und Dianhydroidit;
   - m: 0 oder 1,
   wobei die Reste Z¹, Z², Y¹ bis Y³ gleich oder verschieden sein können und mindestens ein Rest Z¹ oder Z² eine polymerisierbare Gruppe oder einen Rest, der eine polymerisierbare Gruppe enthält, bedeutet,
   oder
b) eine Mischung aus
   b₁) mindestens einem achiralen flüssigkristallinen polymerisierbaren Monomeren der allgemeinen Formel II

      Z³-Y⁶-(A³)ₒ-Y⁷-M¹-Y⁸-(A⁴)ₚ-Y⁹-Z⁴ II

      in der die Variablen unabhängig voneinander folgende Bedeutung haben:
      - A³ und A⁴: ein Spacer, ausgewählt aus der Gruppe, bestehend aus
      -(CH₂)_{q}-, -(CH₂CH₂O)ᵣCH₂CH₂-, -CH₂CH₂SCH₂CH₂-, -CH₂CH₂NHCH₂CH₂-, 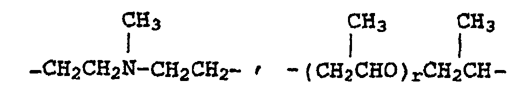 und 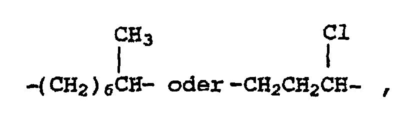 wobei r für 1 bis 3 und q für 1 bis 12 steht;
      - M¹: eine mesogene Gruppe, ausgewählt aus der Gruppe, bestehend aus 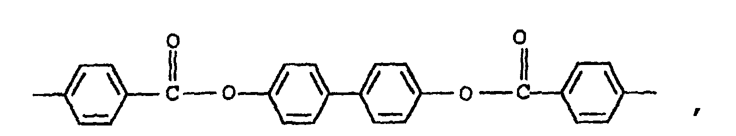 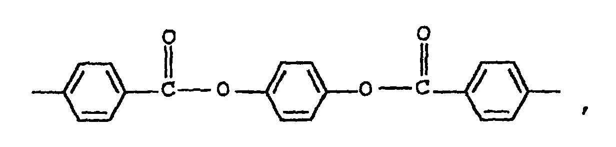 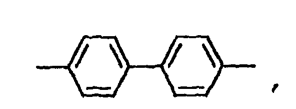 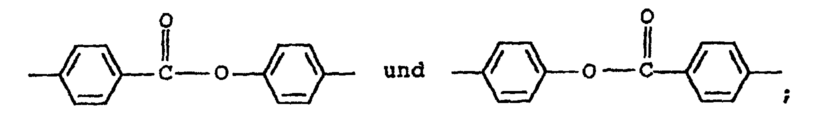
      - Y⁶ bis Y⁹: eine chemische Bindung, -O-, -S-, -C(=O)-, -C(=O)-O-, -O-C(=O)-, -CH=CH-C(=O)-O-, -O-C(=O)-O-, -C(=O)-N(R¹)- oder -(R¹)N-C(=O)-, -CH₂-O-, -O-CH₂-, -CH=N-, -N=CH- oder -N=N-;
      - R¹: Wasserstoff, C₁-C₄-Alkyl;
      - o: 0 oder 1;
      - p: 0 oder 1
      - Z³ und Z⁴: Wasserstoff, C₁-C₄-Alkyl, eine polymerisierbare Gruppe oder ein Rest, der eine polymerisierbare Gruppe trägt, ausgewählt aus
      CH₂=CH- , CH≡C- , 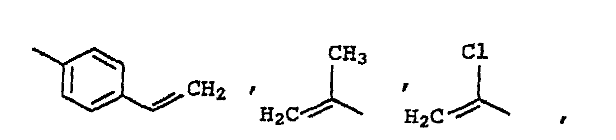 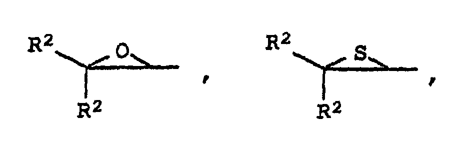 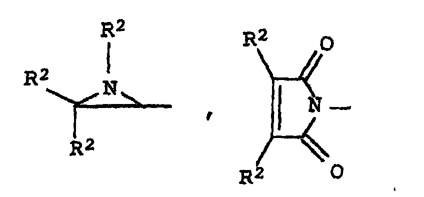 -N=C=O, -N=C=S, -O-C≡N, -COOH, -OH und NH₂,
      wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten und wobei mindestens ein Rest Z³ oder Z⁴ eine polymerisierbare Gruppe oder einen Rest, der eine polymerisierbare Gruppe enthält, und
   b₂₎ mindestens ein chirales flüssigkristallines, polymerisierbares Monomer der allgemeinen Formel I, definiert gemäß Anspruch 1, mit dem eine cholesterisch-flüssigkristalline Phase mit einer Ganghöhe von kleiner 450 nm erzielt werden kann.

Ebenso wie in Formel I ist es auch möglich, die mesogene Gruppe direkt mit den Resten Z³ oder Z⁴ zu verknüpfen. In diesen Fällen stehen o und/oder p für 0 sowie Y⁶ und Y⁷ und/oder Y⁸ und Y⁹ gemeinsam für eine chemische Bindung.

Das Gemisch b) enthält außerdem noch als chiralen Zusatzstoff b₂) mindestens eine Verbindung der bereits oben beschriebenen Formel I.

Geeignete Dotierstoffe sollten eine hohe Verdrillungsfähigkeit besitzen, so daß geringe Mengen des Dotierstoffs zur Induktion der helikalen Struktur ausreichen. Außerdem sollten die chiralen Dotierstoffe eine gute Kompatibilität zu den flüssigkristallinen Verbindungen zeigen, so daß eine effektive Wechselwirkung zwischen diesen Komponenten ermöglicht wird.

Das Ausmaß der Verdrillung hängt jeweils von der Verdrillungsfähigkeit des chiralen Dotierstoffs und von seiner Konzentration ab. Damit hängt also die Ganghöhe der Helix und wiederum auch die Interferenzwellenlänge von der Konzentration des chiralen Dotierstoffs ab. Es kann daher für den Dotierstoff kein allgemeingültiger Konzentrationsbereich angegeben werden. Der Dotierstoff wird in der Menge zugegeben, mit der die gewünschte UV-Reflektion erzielt wird.

Bevorzugte chirale Zusatzstoffe für b₂) sind Verbindungen der Formel Ia 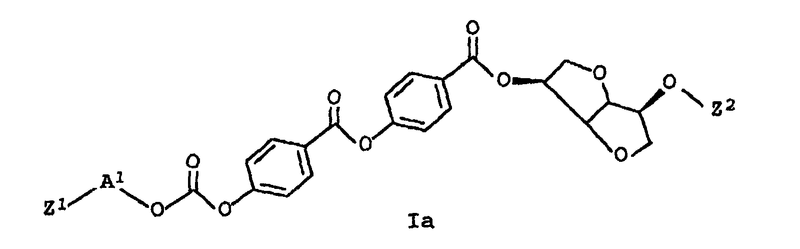 in der die Variablen unabhängig voneinander folgende Bedeutung haben:
- A¹: ein Spacer einer Kettenlänge von 1 bis 6 C-Atomen, bevorzugt ein Spacer, ausgewählt aus der Gruppe, bestehend aus Ethylen, Propylen, n-Butylen, n-Pentylen und n-Hexylen;
- Z¹ und Z²: CH₂=CH-,

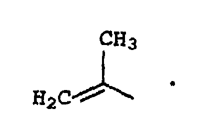

Als besonders bevorzugte Monomere II sind folgende Strukturen zu nennen: 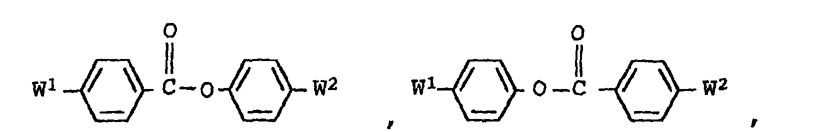 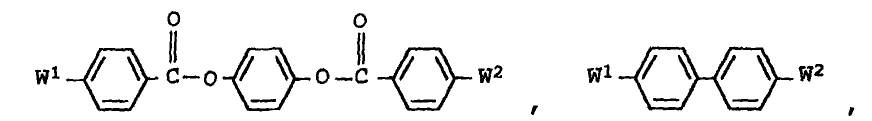 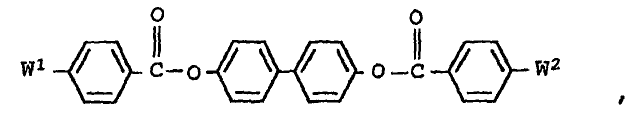 W¹: CH₂=CH-C(=O)-O-(CH₂)₄-O- , W²: -O-(CH₂)₄-O-C(=O)-CH=CH₂ 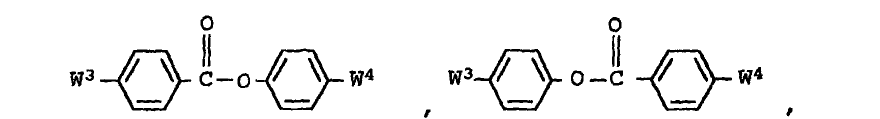 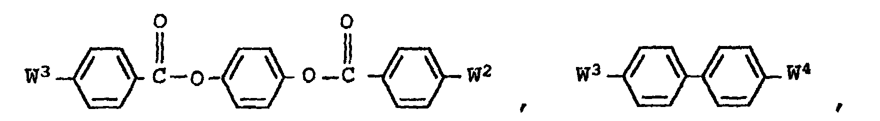 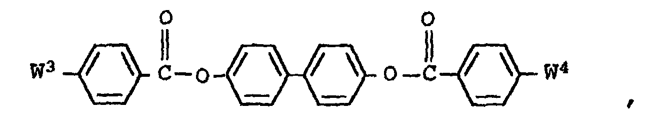 W³: CH₂=CH-C(=O)-O-(CH₂)₆-O- , W⁴: -O-(CH₂)₆-O-C(=O)-CH=CH₂ 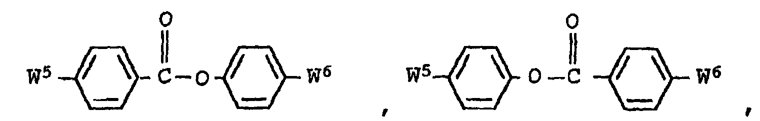 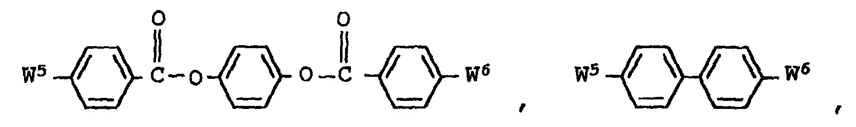 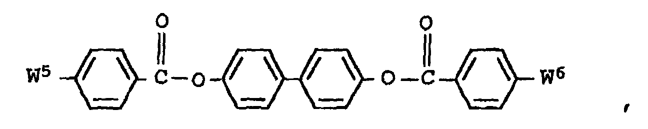 W⁵: CH₂=C(CH₃)-C(=O)-O-(CH₂)₄-O-, W⁶: -O-(CH₂)₄-O-C(=O)-C(CH₃)=CH₂ 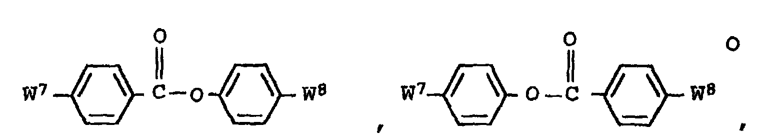 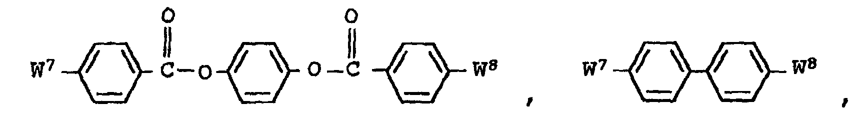 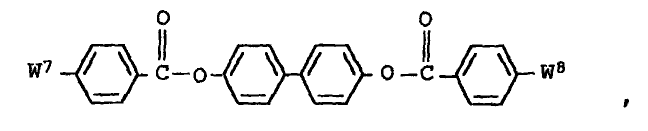 W⁷: CH₂=C(CH₃)-C(=O)-O-(CH₂)₆-O-, W⁸: -O-(CH₂)₆-O-C(=O)-C(CH₃)=CH₂ 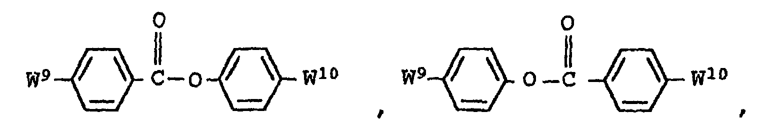 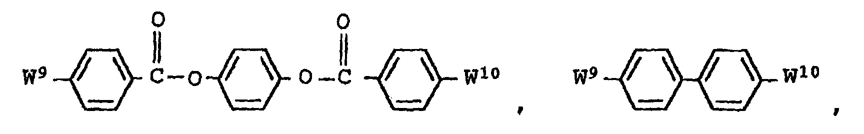 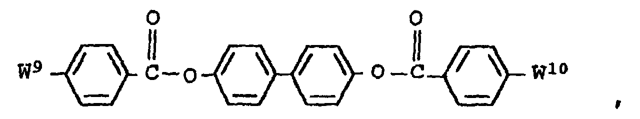 W⁹: CH₂=CH-C(=O)-O-(CH₂)₄-O-C(=O)-O- ,
W¹⁰: -O-(O=)C-O-(CH₂)₄-O-C(=O)-CH=CH₂ 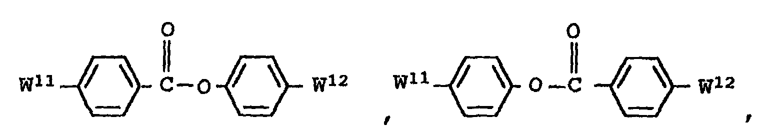 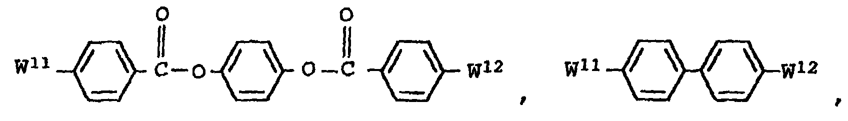 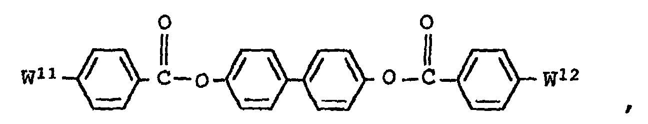 W¹¹: CH₂=CH-C(=O)-O-(CH₂)₆-O-C(=O)-O- ,
W¹²: -O-(O=)C-O-(CH₂)₆-O-C(=O)-CH=CH₂ 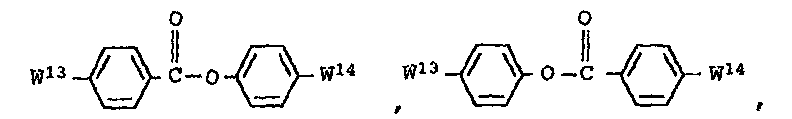 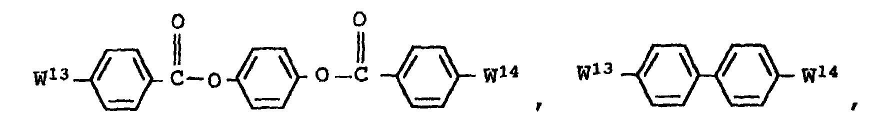 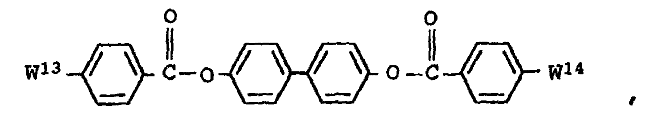 W¹³: CH₂=C(CH₃)-C(=O)-O-(CH₂)₄-O-C(=O)-O- ,
W¹⁴: -O-(O=)C-O-(CH₂)₄-O-C(=O)-C(CH₃)=CH₂ 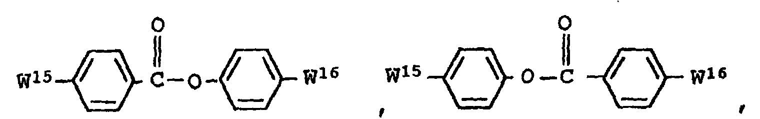 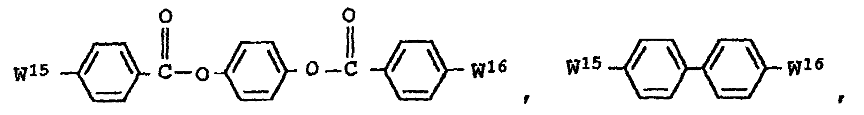 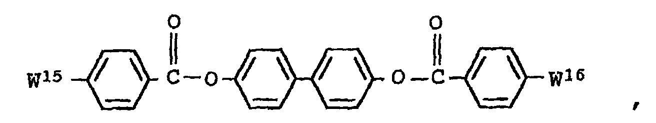 W¹⁵: CH₂=C(CH₃)-C(=O)-O-(CH₂)₆-O-C(=O)-O- ,
W¹⁶: -O-(O=)C-O-(CH₂)₆-O-C(=O)-C(CH₃)=CH₂

Als besonders bevorzugte Monomere I sind folgende, Dianhydromannit als zentralen Baustein enthaltende Strukturen zu nennen: 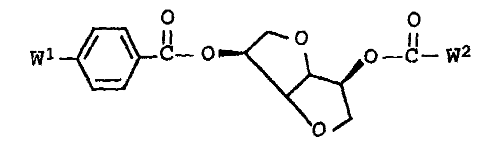 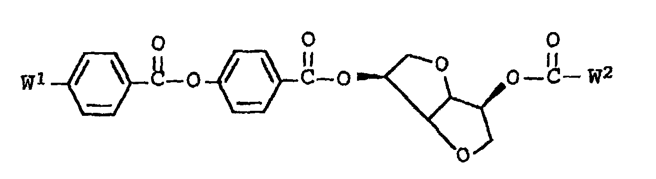 W¹: CH₂=CH-C(=O)-O-(CH₂)₄-O- W²: -O-(CH₂)₄-O-C(=O)-CH=CH₂ 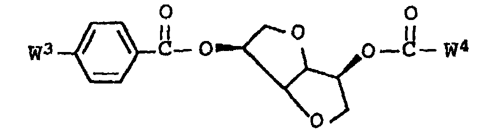 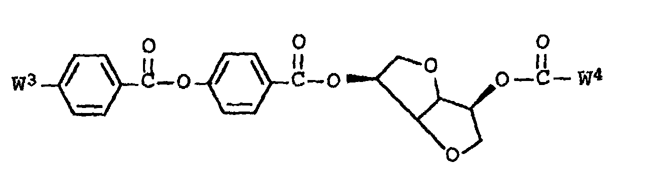 W³: CH₂=CH-C(=O)-O-(CH₂)₆-O- W⁴: -O-(CH₂)₆-O-C(=O)-CH=CH₂ 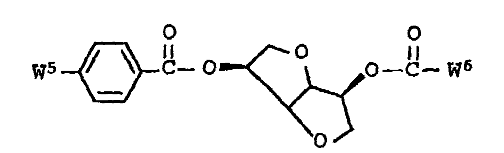 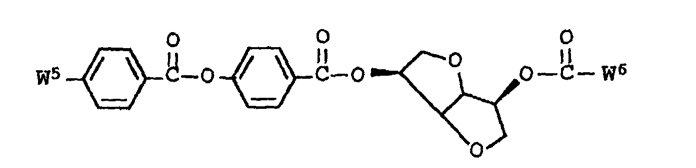 W⁵: CH₂=C(CH₃)-C(=O)-O-(CH₂)₄-O-, W⁶: -O-(CH₂)₄-O-C(=O)-C(CH₃)=CH₂ 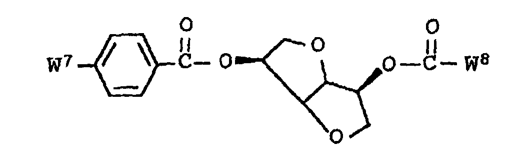 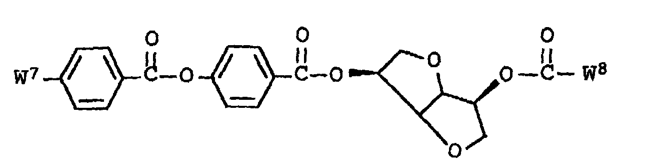 W⁷: CH₂=C(CH₃)-C(=O)-O-(CH₂)₆-O-, W⁸: -O-(CH₂)₆-O-C(=O)-C(CH₃)=CH₂ 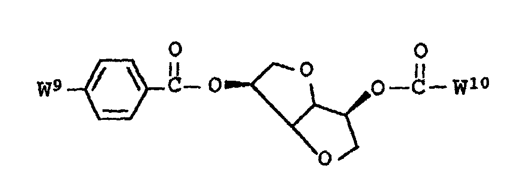 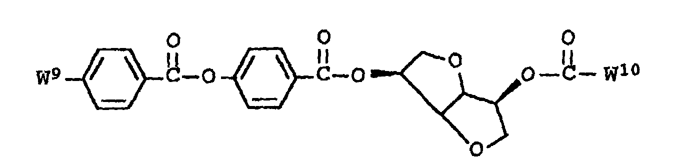 W⁹: CH₂=CH-C(=O)-O-(CH₂)₄-O-C(=O)-O-
W¹⁰: -O-(O=)C-O-(CH₂)₄-O-C(=O)-CH=CH₂ 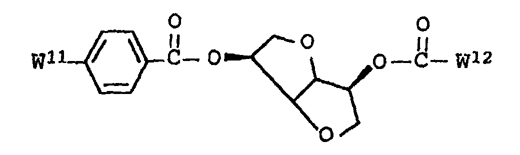 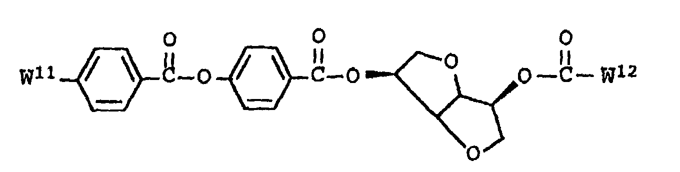 W¹¹: CH₂=CH-C(=O)-O-(CH₂)₆-O-C(=O)-O-
W¹²: -O-(O=)C-O-(CH₂)₆-O-C(=O)-CH=CH₂ 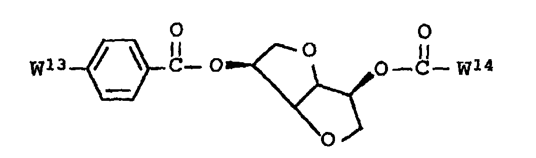 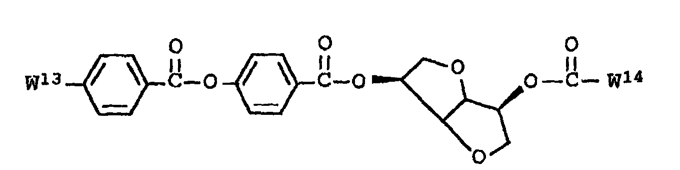 W¹³: CH₂=C(CH₃)-C(=O)-O-(CH₂)₄-O-C(=O)-O-,
W¹⁴: -O-(O=)C-O-(CH₂)₄-O-C(=O)-C(CH₃)=CH₂ 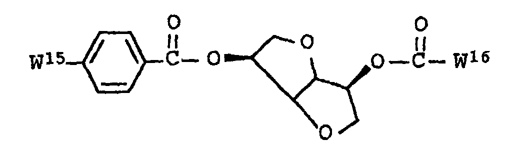 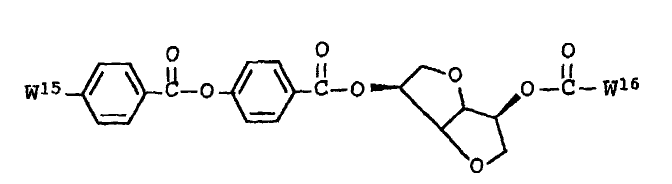 W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂

Die Gewichtsverhältnisse von Komponente II zu Komponente I liegen im Bereich von 99 zu 1 bis 40 zu 60, bevorzugt im Bereich von 99 zu 1 bis 70 zu 30, besonders bevorzugt von 98 zu 2 bis 85 zu 15.

Verwendung der erfindungsgemäßen cholesterisch-flüssigkristallinen Zusammensetzungen in kosmetischen und pharmazeutischen Zubereitungen:

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welahe sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Dermatologische Untersuchungen haben gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Neben den bekannten UV-Absorbern, wie z.B. 4-Methoxy-zimtsäure-2-ethylhexylester oder 3-(4'-Methyl)-benzyliden-bornan-2-on werden in kosmetischen und pharmazeutischen Formulierugen häufig auch Lichtschutzmittel eingesetzt, die in Form von Pigmenten die UV-Strahlen reflektieren bzw. absorbieren. Die wichtigsten dieser verwendeten Pigmente sind Titandioxid und Zinkoxid. Bei hohen Einsatzkonzentrationen kann mit Pigmenten eine vollständige Abdeckung der Haut erreicht werden. Dann reflektieren die Partikel allerdings nicht nur UV-Strahlung sondern auch sichtbares Licht, was die häufig nicht gewünschte starke Eigenfärbung pigmenthaltiger Präparate bewirkt.

Während grobteilige Titandioxid Pigmente (Teilchengröße > 500 nm) im UV-B- und UV-A-Bereich vergleichbar wirken, verschiebt sich das Wirkungsspektrum bei feinteiligem Material mit abnehmender Teilchengröße in Richtung UV-B. Dies zeigt, daß die Absorptions-/Reflektionscharakteristik direkt von der Größe und der Verteilung der Teilchen abhängt. Für einen ausgewogenen UV-B- und UV-A-Schutz sind daher bestimmte Teilchengrößenverteilungen erforderlich.
Von Nachteil bei der Verwendung der oben genannten Pigmente erweist sich, daß während der Lagerung der kosmetischen oder pharmazeutischen Lichtschutzmittelformulierungen oftmals Agglomeration, Aggregation und/oder Separation der Pigmentteilchen stattfinden. Die Folge der hierdurch veränderten optischen Eigenschaften kann eine drastisch verringerte Lichtschutzwirkung sein.

Als Alternative zu den o.g. Pigmenten beschreibt DE-A-196 19 460 die Verwendung von Flüssigkristallmischungen mit cholesterischer Phase enthaltend a) flüssigkristalline Organosiloxane, die Dianhydrohexit-Derivate als chirale Gruppen aufweisen und b) chirale monomere Zusatzstoffe, die die gleiche Helizität induzieren wie die jeweiligen flüssigkristallinen Organosiloxane, zur Herstellung von, für kosmetische Zwecke geeignete UV-Schutzschichten in Form von Filmen oder Plättchen. Die hier beschriebenen Flüssigkristallmischungen haben den Nachteil, daß sie sich aufgrund ihrer hohen Viskosität nur unbefriedigend zu Pigmenten verarbeiten lassen.

DE-A-196 29 761 beschreibt kosmetische oder pharmazeutische Zubereitungen, enthaltend Pigmente aus Polyorganosiloxanen mit vom Betrachtungswinkel abhängiger Farbigkeit. Bei den Pigmenten handelt es sich dabei um mindestens eine orientierte vernetzte Substanz einer flüssigkristallinen Struktur mit chiraler Phase. Die hier in den kosmetischen und pharmazeutischen Rezepturen offenbarten Pigmente besitzen zwar gewisse Absorptionseigenschaften im UV-Bereich. Sie haben jedoch für bestimmte Anwendungen den Nachteil, daß es sich hierbei um farbige Verbindungen handelt deren Einsatzgebiet dadurch eingeschränkt ist. Sehr häufig sind aber gerade solche kosmetischen und pharmazeutischen Zubereitungen gefragt, mit denen ein UV-Schutz erzielt wird, bei denen aber eine Färbung der Zubereitung unerwünscht ist.

Gegenstand der Erfindung ist daher auch die Verwendung der o.g. cholesterisch-flüssigkristallinen Zusammensetzungen als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

Die für die Verwendung als UV-Filter in kosmetischen und pharmazeutischen Zubereitungen bevorzugt verwendeten cholesterisch-flüssigkristallinen Zusammensetzungen enthalten eine Mischung aus mindestens einem achiralen flüssigkristallinen polymerisierbaren Monomeren der Formel II und mindestens einem chiralen polymerisierbaren Monomeren der Formel I.

Für die erfindungsgemäße Verwendung der oben genannten cholesterisch-flüssigkristallinen Zusammensetzungen a) und b) als UV-Filter in kosmetischen und pharmazeutischen Zubereitungen können die in diesen Zusammensetzungen enthaltenen Komponenten der Formel I und II direkt in die kosmetischen und pharmazeutischen Zubereitungen eingearbeitet werden.

Bevorzugt werden jedoch die erfindungsgemäß verwendeten cholesterisch-flüssigkristallinen Zusammensetzungen in Form von Pigmenten eingesetzt. Diese Pigmente sind dadurch erhältlich, daß die in den cholesterisch-flüssigkristallinen Zusammensetzungen enthaltenen Monomeren I und II mit Hilfe ihrer polymerisierbaren Gruppen durch radikalische oder ionische Polymerisationsverfahren, welche durch eine photochemische Reaktion gestartet werden können, in hochvernetzte Polymere mit eingefrorener flüssigkristalliner Ordnungsstruktur überführt werden.

Die Herstellung solcher Pigmente ist bekannt und wird u.a. ausführlich in der deutschen Anmeldung P 19738369.6 beschrieben.

Einen Überblick über Verfahren, orientierte Ausgangsstoffe photochemisch zu vernetzen, findet sich darüberhinaus bei C. G. Roffey, Photopolymerisation of Surface Coatings, (1982) John Willey & Sons, Chichester, S. 137 bis 208.

In einer bevorzugten Ausführungsform werden die dreidimensional vernetzbaren polymerisierbaren Monomeren auf eine Unterlage aufgebracht, auf dieser Unterlage vernetzt und nach dem Vernetzen von der Unterlage abgelöst.

Die zu einem Film vernetzten cholesterisch-flüssigkristallinen Zusammensetzungen lassen sich nach der Polymerisation durch Mahlung auf die jeweils erwünschte Korngröße zerkleinern. Je nach der erwünschten Anwendung bzw. je nach Art der kosmetischen oder pharmazeutischen Formulierung können Korngrößen mit einem Durchmesser von 1 bis 1000 µm hergestellt werden. Bevorzugte Korngrößen liegen im Bereich zwischen 1 und 100 µm, besonders bevorzugt zwischen 15 und 50 µm.

Die Dicke der Pigmente liegt zwischen 1 und 100 µm, bevorzugt zwischen 1 und 50 µm besonders bevorzugt zwischen 1,5 und 10 µm.

Die cholesterisch-flüssigkristallinen Zusammensetzungen a) bzw. b), die als Ausgangssubstanzen zur Herstellung der Pigmente geeignet sind, besitzen eine verdrillte Struktur mit einer Ganghöhe, die einer Wellenlänge des Lichtes bis zu 450 nm entspricht. Wie in der bevorzugten Ausführungsform b) gezeigt wird, können sich diese verdrillten Strukturen einer definierten Ganghöhe aus nematischen Strukturen b₁) erhalten werden, indem man ihnen eine chirale Substanz b₂) zusetzt. Art und Anteil der chiralen Substanz bestimmen die Ganghöhe der verdrillten Struktur und damit die Wellenlänge des reflektierten Lichtes. Je nach Chiralität der eingesetzten optisch aktiven Zusatzstoffe kann die Verdrillung der Struktur sowohl links- als auch rechtsgängig sein.

Sogenannte Breitbandreflektoren lassen sich durch einfaches Mischen mehrerer der erfindungsgemäß zu verwendenden cholesterisch flüssigkristallinen Pigmente mit jeweils unterschiedlichen UV-Reflektionsmaxima erzeugen.

Darüber hinaus ist es möglich, durch Mischen von mindestens zwei verschiedenen Pigmenten der cholesterisch-flüssigkristallinen Zusammensetzungen a) und/oder b) mit jeweils entgegengesetzter Verdrillung (Helicität) eine vollständige Reflektion der UV-Strahlen zu erzielen. Pigmente solcher jeweils entgegengesetzt verdrillten, cholesterisch-flüssigkristallinen Strukturen sind beispielsweise durch Zugabe jeweils der einzelnen Spiegelbildisomeren (Enantiomeren) oder Diastereomeren der chiralen Zusatzstoffe b₂) zu dem achiralen flüssigkristallinen polymerisierbaren Monomeren b₁) erhältlich. Die Ganghöhe der jeweils entgegengesetzt verdrillten Strukturen kann dabei gleich oder verschieden sein.

Es ist auch möglich, zunächst die cholesterisch-flüssigkristalline Zusammensetzungen a) oder b) jeweils von entgegengesetzter Verdrillung zu mischen, diese anschließend durch o.g. Vernetzung in die bereits beschriebenen Pigmente zu überführen und als UV-Reflektoren in kosmetischen und pharmazeutischen Formulierungen einzusetzen.

Neben den o.g. Mischungen cholesterisch flüssigkristalliner Pigmente ist es auch möglich Mehrschichtpigmente zu erzeugen, deren einzelne Schichten verschiedene, erfindungsgemäß zu verwendende, dreidimensional vernetzte cholesterisch flüssigkristalline Zusammensetzungen enthalten. Das Design derartiger Mehrschichtpigmente läßt sich vielfältig variieren. So können u.a.
- einzelne Schichten von vernetzten cholesterisch flüssigkristallinen Zusammensetzungen entgegengesetzter Verdrillung oder
- einzelne Schichten von vernetzten cholesterisch flüssigkristallinen Zusammensetzungen gleicher Gangrichtung aber unterschiedlicher Ganghöhe und somit unterschiedlicher Reflektionseigenschaften
übereinander aufgebracht werden.

Bevorzugt sind sogenannte Dreisohichtpigmente, bei denen die beiden äußeren Schichten aus jeweils mindestens einer der erfindungsgemäß zu verwendenden vernetzten, cholesterisch flüssigkristallinen Zusammensetzungen besteht und die mittlere Schicht beispielsweise eine Bindemittelmatrix enthalten kann, in dem zusätzlich ein weiterer UV-Absorber eingearbeitet sein kann. Einzelheiten bezüglich Herstellung, Eigenschaften und weiterer Bestandteile solcher mehrschichtigen cholesterischen Pigmente sind der deutschen Patentanmeldung P 19738368.8 zu entnehmen.

Gegenstand der Erfindung sind somit auch die oben beschriebenen Pigmente, insbesondere Mehrschichtpigmente, enthaltend die eingangs genannten cholesterisch-flüssigkristallinen Zusammensetzungen.

Ein Vorteil der erfindungsgemäß verwendeten Pigmente liegt darin, daß deren Zusammensetzung so maßgeschneidert eingestellt werden kann, damit mit diesen Pigmenten die gewünschte UV-Reflektion erzielt werden kann ohne aber eine eigene Farbigkeit (im sichtbaren Bereich) zu zeigen.

Ein weiterer Vorteil der Pigmente liegt in ihren physikalischen Eigenschaften. Aufgrund ihrer geringen Dichte (im Vergleich beispielsweise zu TiO₂) lassen sich die Pigmente gut in Emulsionen einarbeiten, ohne daß es zu Aggregation oder Separation der Pigmentteilchen kommt.

Die erfindungsgemäß zu verwendenden Pigmente lassen sich durch einfaches Abmischen in die kosmetischen und pharmazeutischen Zubereitungen einarbeiten.

Gegenstand der vorliegenden Erfindung sind weiterhin kosmetische und pharmazeutische Zubereitungen, die 0,1 bis 20 Gew.-%, vorzugsweise 0,5 bis 10 Gew.-%, besonders bevorzugt 1 bis 7 Gew.-%, bezogen auf die gesamte Menge der kosmetischen und pharmazeutischen Zubereitung, eine oder mehrere der cholesterisch-flüssigkristallinen Zusammensetzungen aus a) mindestens einem chiralen flüssigkristallinen, polymerisierbaren Monomeren der allgemeinen Formel I [Z¹-Y¹-(A¹)ₘ-Y²-M-Y³-X-Y⁴-(A²)ₙ-Y⁵-Z²] mit dem eine cholesterisch-flüssigkristalline Phase mit einer Ganghöhe von kleiner 450 nm erzielt werden kann oder b) einer Mischung aus mindestens einem achiralen flüssigkristallinen polymerisierbaren Monomeren der allgemeinen Formel II [Z³-Y⁶-(A³)ₒ-Y⁷-M¹-Y⁸-(A⁴)ₚ-Y⁹-Z⁴] und mindestens einem chiralen flüssigkristallinen, polymerisierbaren Monomeren der allgemeinen Formel I mit der eine cholesterisch-flüssigkristalline Phase mit einer Ganghöhe von kleiner 450 nm erzielt werden kann, zusammen mit an sich für kosmetische und pharmazeutische Zubereitungen bekannten, im UV-A- und UV-B-Bereich absorbierenden Verbindungen als Lichtschutzmittel enthalten. Die Variablen der Formeln I und II sowie die Stoffklasse der eingesetzten chiralen Zusatzstoffe entsprechen dabei sowohl in ihrer allgemeinen als auch in ihrer bevorzugten Ausführungsform den bereits oben geschilderten Erläuterungen.

Bevorzugt sind solche der oben genannten kosmetischen und pharmazeutischen Zubereitungen, die die erfindungsgemäß zu verwendenden cholesterisch-flüssigkristallinen Zusammensetzungen in Form der bereits beschriebenen Pigmente, insbesondere in Form von mehrschichtigen Pigmenten enthalten.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Bthylhexansäurestearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen.

Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Die erfindungsgemäßen Zubereitungen enthalten vorteilhaft ein oder mehrere Antioxidantien. Als günstige, aber dennoch fakultativ zu verwendende Antioxidantien können alle für kosmetische und/oder dermatologische Anwendungen geeigneten oder gebräuchlichen natürlichen, synthetischen und/oder partialsynthetischen Antioxidantien verwendet werden.

Besonders vorteilhaft werden die Antioxidantien gewählt aus der Gruppe, bestehend aus:

Aminosäuren (z.B. Glycin, Histidin, Tyrosin, Tryptophan) und deren Derivate, Imidazole (z.B. Urocaninsäure) und deren Derivate, Peptide wie D,L,-Carnosin, D-Carnosin, L-Carnosin und deren Derivate (z.B. Anserin), Carotinoide (z.B. β-Carotin, Lycopin) und deren Derivate, Liponsäure und deren Derivate (z.B. Dihydroliponsäure), Aurothioglucose, Propylthiouracil und andere Thioverbindungen (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA, EGTA und deren Derivate, ungesättigte Fettsäuren und deren Derivate (z.B. γ-Linolensäure, Linolsäure, ölsäure), Folsäure und deren Derivate (z.B. 5-Methyltetrahydrofolsäure), Ubichinon und Ubichinol und deren Derivate, Vitamin C und deren Derivate (z.B. Ascorbylpalmitat, Ascorbylphosphate, Ascorbylacetate), Tocopherole und Derivate (z.B. Tocopherylacetat, Tocotrienol), Vitamin A und Derivate (z.B. Vitamin A-Palmitat), Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Stilbene und deren Derivate.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Zubereitung - betragen. Die Herstellung der Zubereitung kann in an sich bekannter Weise, d.h. beispielsweise durch Heiß-, Kalt-, Heiß-/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden cholesterisch-flüssigkristallinen Zusammensetzungen 10 bis 90 Gew.-%, bevorzugt 20 bis 70 Gew.-% bezogen auf die Gesamtmenge von UV-B und UV-A absorbierenden Substanzen.

Als UV-Filtersubstanzen, die in Kombination mit den erfindungsgemäß zu verwendenden cholesterisch-flüssigkristallinen Zusammensetzungen angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |

Schließlich sind auch mikronisierte Pigmente wie Titandioxid und Zinkoxid zu nennen.

Zum Schutz menschlicher Haare vor UV-Strahlen können die erfindugsgemäß verwendeten cholesterisch flüssigkristallinen Zusammensetzungen a) und/oder b) in Shampoos, Lotionen, Gelen, Haarsprays, Aerosol-Schaumcremes oder Emulsionen in Konzentrationen von 0,1 bis 20 Gew.-%, bevorzugt 0,5 bis 10 Gew.-%, besonders bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Zusammensetzungen zeichnen sich in der Regel durch ein besonders hohes Reflektionsvermögen im Bereich der UV-A- und UV-B-Strahlung mit scharfer Bandenstruktur aus. Weiterhin lassen sie sich leicht in kosmetische und pharmazeutischen Formulierungen einarbeiten. Außerdem zeichnen sie sich besonders durch ihre hohe Photostabilität, und die damit hergestellten Zubereitungen durch ihr angenehmes Hautgefühl aus.

Die UV-Filterwirkung der erfindungsgemäßen verwendeten cholesterisch flüssigkristallinen Zusammensetzungen a) und/oder b) kann auch zur Stabilisierung von Wirk- und Hilfsstoffen in kosmetischen und pharmazeutischen Formulierungen ausgenutzt werden.

Die folgenden Beispiele sollen die erfinderische Verwendung der cholesterisch flüssigkristallinen Zusammensetzungen näher erläutern.

### Beispiel 1

Herstellung des chiralen Dotierstoffs der Formel Ib

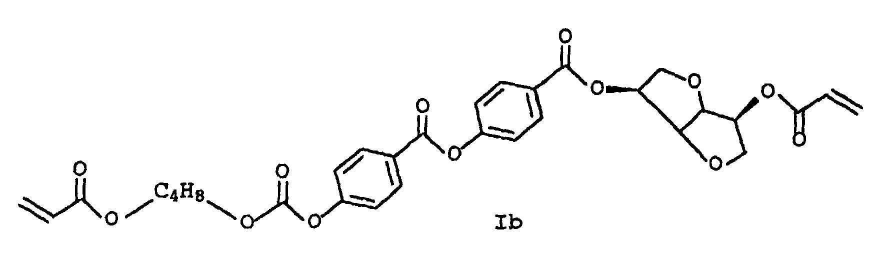

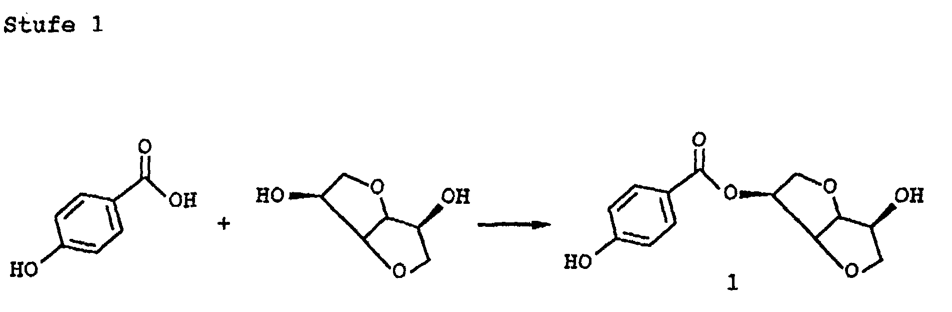

298 g (2,0 mol) Isomannit, 276 g (2,0 mol) 4-Hydroxybenzoesäure und 16 g p-Toluolsulfonsäure wurden in 1 l Xylol gelöst und 3,5 h unter Rückfluß und Auskreisen von Wasser erhitzt. Anschließend wurden 2 l Wasser/Methanol (1:1) zur azeotropen Abtrennung von Xylol zugetropft. Nach Zugabe von weiteren 2 l Wasser und Abkühlung auf Raumtemperatur kristallisierte das Produkt aus. Nach Filtration, Umkristallisation aus Isobutanol und anschließender chromatographischer Aufreinigung erhielt man 39,2 g der Verbindung 1.

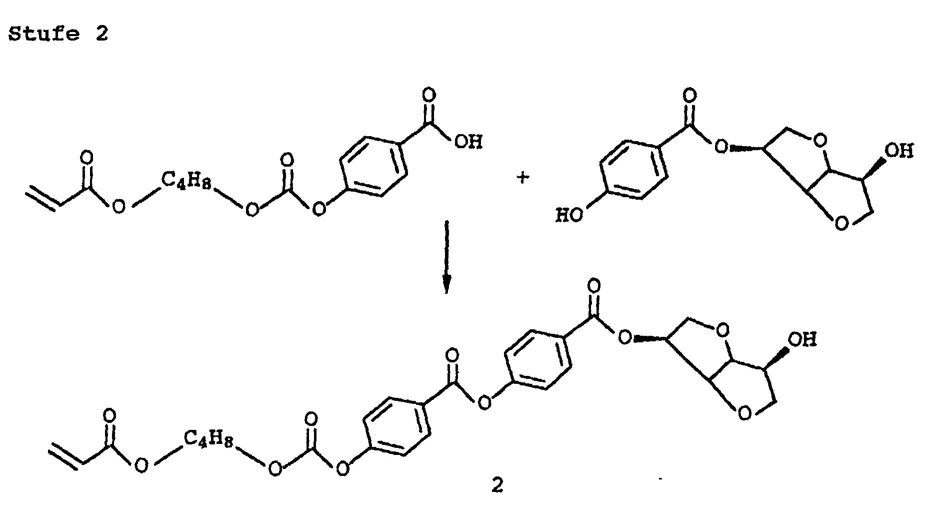

Ein Gemisch aus 28,2 g (0,106 mol) monoacyliertes Isomannit der Formel 1, hergestellt in der Stufe 1 und 36,96 g (0,12 mol) 4-Acryloxybutyloxycarbonyloxybenzoesäure in 150 ml Dichlormethan wurden bei 0 bis 5°C mit 0,1 g 4-Dimethylaminopyridin und stufenweise über 1 h mit 24,72 g Dicyclohexylcarbodiimid versetzt. Die Reaktionsmischung wurde 2,5 h bei 0 bis 5°C und 12 h bei RT gerührt, abgesaugt und das eingeeengte Filtrat chromatographisch an Kieselgel aufgereinigt. Man erhielt 45,9 g der Verbindung 2.

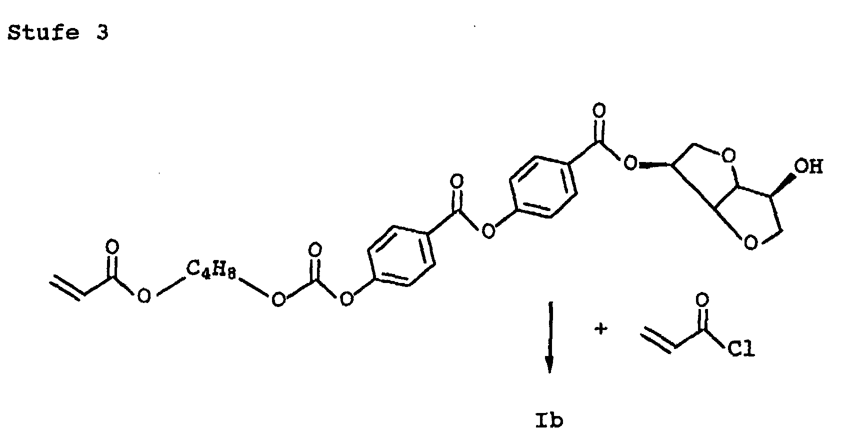

34,89 g (0,056 mol) des Produkts aus Stufe 2 wurden in 120 ml Dichlormethan zusammen mit 27,7 ml (0,2 mol) Triethylamin gelöst und bei 0 bis 5°C mit 9,53 ml (0,12 mol) Acrylsäurechlorid, gelöst in 50 ml Dichlormethan, versetzt. Nach 2 h Nachrührzeit bei 0 bis 5°C und 12 h bei RT wurde das Reaktionsgemisch auf verdünnte Salzsäure gegossen und die organische Phase mit Wasser gewaschen. Nach Trocknung der organischen Phase über Natriumsulfat wurde die Lösung mit Uvinul® FK 4245 (Fa. BASF AG) inhibiert und das Produkt über Kieselgel chromatographisch auf gereinigt. Man erhielt 18,4 g der Verbindung Ib.

## Patentansprüche

1. Chirale Dotierstoffe der allgemeinen Formel I,
Z¹-Y¹-(A¹)ₘ-Y²-M-Y³-X-Z² I
in der die Variablen unabhängig voneinander folgende Bedeutung haben:
A1 ein Spacer, ausgewählt aus der Gruppe, bestehend aus
-(CH₂)_{q}-, -(CH₂CH₂O)ᵣCH₂CH₂-, -CH₂CH₂SCH₂CH₂-, -CH₂CH₂NHCH₂CH₂-, 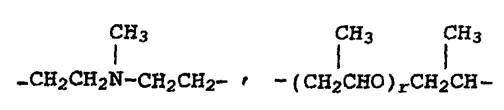 und 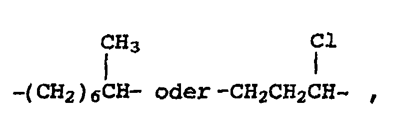 wobei r für 1 bis 3 und q für 1 bis 12 steht.
Y¹ bis Y³ eine chemische Bindung, -O-, -S-, -C(=O)-, -C(=O)-O-, -O-C(=O)-, -CH=CH-C(=O)-O-, -O-C(=O)-O-, -C(=O)-N(R)-oder -(R)N-C(=O)-, -CH₂-O-, -O-CH₂-, -CH=N-, -N=CH- oder -N=N-;
M eine mesogene Gruppe, ausgewählt aus der Gruppe, bestehend aus 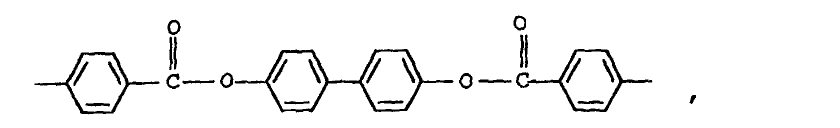 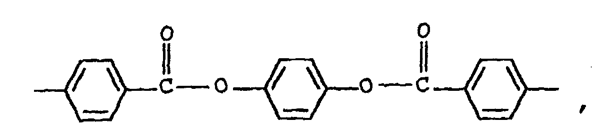 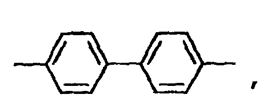 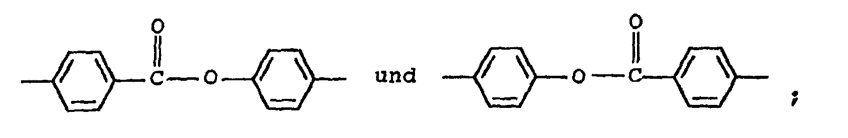
R Wasserstoff, C₁-C₄-Alkyl;
Z¹ und Z² Wasserstoff, C₁-C₄-Alkyl, eine polymerisierbare Gruppe oder ein Rest, der eine polymerisierbare Gruppe trägt, ausgewählt aus
CH₂=CH- , CH≡C- , 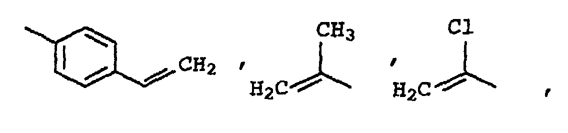 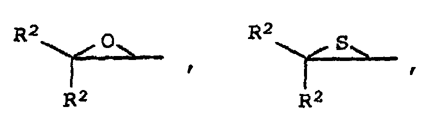 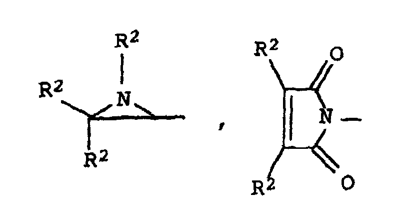 -N=C=O, -N=C=S, -O-C≡N, -COOH, -OH und NH₂,
wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten;
X ein Dianhydrohexitrest, ausgewählt aus der Gruppe, bestehend aus Dianhydrosorbit, Dianhydromannit und Dianhydroidit;
m 0 oder 1,
wobei die Reste Z¹, Z², Y¹ bis Y³ gleich oder verschieden sein können und mindestens ein Rest Z¹ oder Z² eine polymerisierbare Gruppe oder einen Rest, der eine polymerisierbare Gruppe enthält, bedeutet.

2. Chirale Dotierstoffe nach Anspruch 1, in der die Variablen unabhängig voneinander folgende Bedeutung haben:
A¹ ein Spacer, ausgewählt aus der Gruppe, bestehend aus Ethylen, Propylen, n-Butylen, n-Pentylen und n-Hexylen;
Y¹ bis Y³ eine chemische Bindung, -O-, -S-, -C(=O)-, -C(=O)-O-, -O-C(=O)-;
M ein mesogener Rest aus der Gruppe, bestehend aus 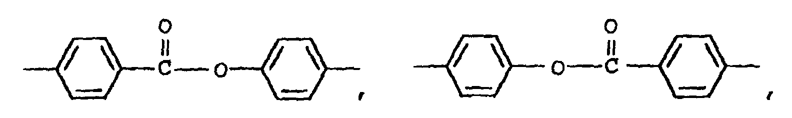 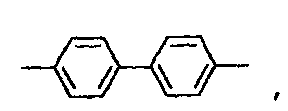 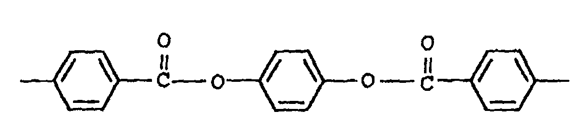 und 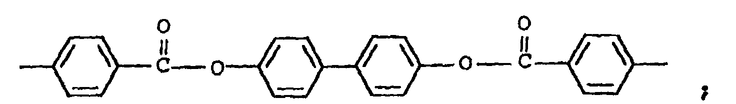
Z¹ und Z² CH₂=CH-, 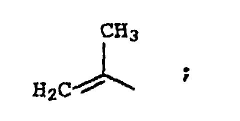
X ein Dianhydromannitrest;
m 0 oder 1.

3. Chirale Dotierstoffe nach Anspruch 1 der allgemeinen Formel Ia, 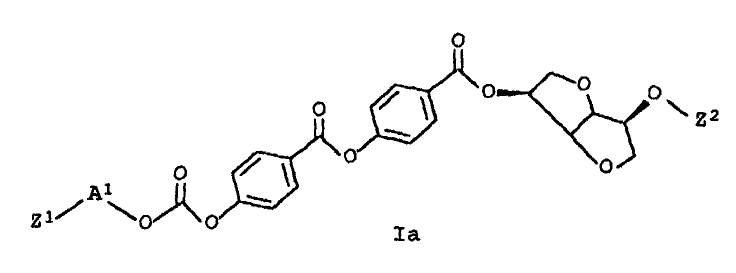 in der die Variablen unabhängig voneinander folgende Bedeutung haben:
A¹ ein Spacer, ausgewählt aus der Gruppe, bestehend aus Ethylen, Propylen, n-Butylen, n-Pentylen und n-Hexylen;
Z¹ und Z² CH₂=CH-,

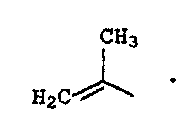

4. Cholesterisch-flüssigkristalline Zusammensetzungen, enthaltend
a) mindestens ein chirales flüssigkristallines, polymerisierbares Monomeres der allgemeinen Formel I,
Z¹-Y¹-(A¹)ₘ-Y²-M-Y³-X-Z² I
definiert gemäß Anspruch 1,
mit dem eine cholesterisch-flüssigkristalline Phase mit einer Ganghöhe von kleiner 450 nm erzielt werden kann, in der die Variablen die in Anspruch 1 genannte Bedeutung haben
oder
b) eine Mischung aus
b₁) mindestens einem achiralen flüssigkristallinen polymerisierbaren Monomeren der allgemeinen Formel II
Z³-Y⁶-(A³)ₒ-Y⁷-M¹-Y⁸-(A⁴)ₚ-Y⁹-Z⁴ II
in der die Variablen unabhängig voneinander folgende Bedeutung haben:
A³ und A⁴ ein Spacer, ausgewählt aus der Gruppe, bestehend aus
-(CH₂)_{q}-, -(CH₂CH₂O)ᵣCH₂CH₂-, -CH₂CH₂SCH₂CH₂-, -CH₂CH₂NHCH₂CH₂-, 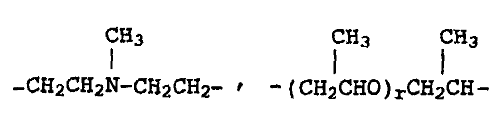 und 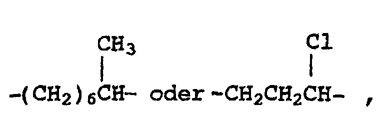 wobei r für 1 bis 3 und q für 1 bis 12 steht;
M¹ eine mesogene Gruppe, ausgewählt aus der Gruppe, bestehend aus 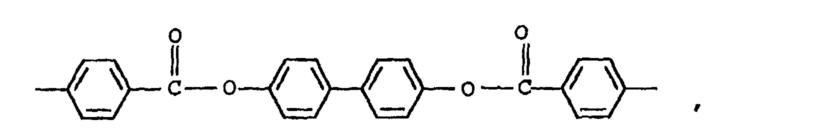 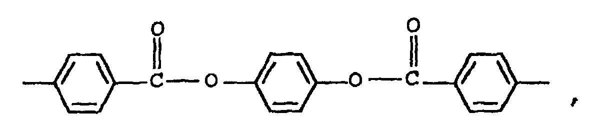 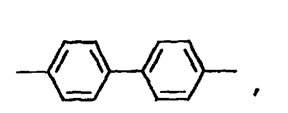 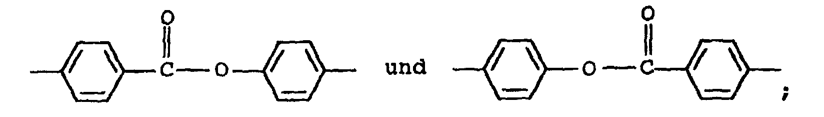
Y⁶ bis Y⁹ eine chemische Bindung, -O-, -S-, -C(=O)-, -C(=O)-O-, -O-C(=O)-, -CH=CH-C(=O)-O-, -O-C(=O)-O-, -C(=O)-N(R¹)- oder -(R¹)N-C(=O)-, -CH₂-O-, -O-CH₂-, -CH=N-, -N=CH- oder -N=N-;
R¹ Wasserstoff, C₁-C₄-Alkyl;
o 0 oder 1;
p 0 oder 1
Z³ und Z⁴ Wasserstoff, C₁-C₄-Alkyl, eine polymerisierbare Gruppe oder ein Rest, der eine polymerisierbare Gruppe trägt, ausgewählt aus
CH₂=CH- , CH≡C- , 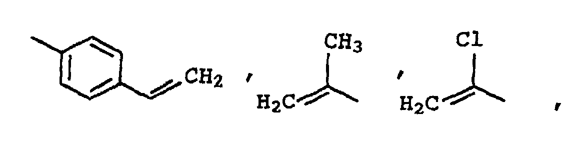 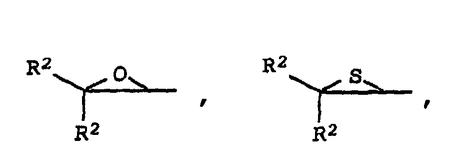 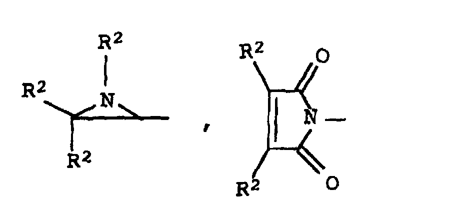 -N=C=O, -N=C=S, -O-C≡N, -COOH, -OH und NH₂,
wobei die Reste R² gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl bedeuten und wobei mindestens ein Rest Z³ oder Z⁴ eine polymerisierbare Gruppe oder einen Rest, der eine polymerisierbare Gruppe enthält, und
b₂₎ mindestens ein chirales flüssigkristallines, polymerisierbares Monomer der allgemeinen Formel I, definiert gemäß Anspruch 1, mit dem eine cholesterisch-flüssigkristalline Phase mit einer Ganghöhe von kleiner 450 nm erzielt werden kann.

5. Cholesterisch-flüssigkristalline Zusammensetzungen nach Anspruch 4, enthaltend als chirales flüssigkristallines, polymerisierbares Monomer a) oder b₂) mindestens eine chirale Verbindung der allgemeinen Formel Ia, 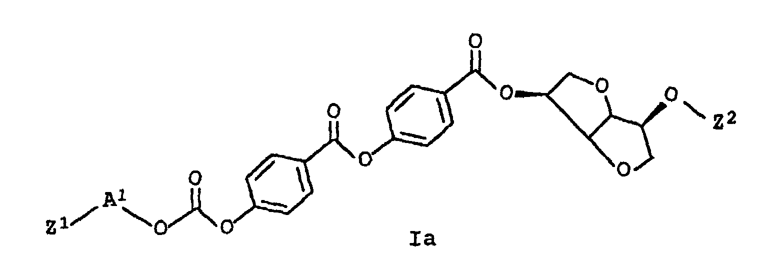 in der die Variablen unabhängig voneinander folgende Bedeutung haben:
A¹ ein Spacer, ausgewählt aus der Gruppe, bestehend aus Ethylen, Propylen, n-Butylen, n-Pentylen und n-Hexylen;
Z¹ und Z² CH₂=CH-,

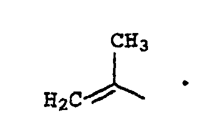

6. Cholesterisch-flüssigkristalline Zusammensetzungen nach einem der Ansprüche 4 oder 5, **dadurch gekennzeichnet, daß** sie im UV-Bereich linkszirkular polarisiertes Licht reflektieren.

7. Verwendung von cholesterisch-flüssigkristallinen Zusammensetzungen, definiert gemäß einem der Ansprüche 4 bis 6, als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

8. Verwendung von cholesterisch-flüssigkristallinen Zusammensetzungen nach Anspruch 7 als photostabile UV-Reflektoren.

9. Verwendung von cholesterisch-flüssigkristallinen Zusammensetzungen, definiert gemäß einem der Ansprüche 4 bis 6, als UV-Stabilisatoren in kosmetischen und pharmazeutischen Formulierungen.

10. Verwendung von cholesterisch-flüssigkristallinen Zusammensetzungen nach einem der Ansprüchen 7 bis 9 in Form von Pigmenten.

11. Pigmente enthaltend polymerisierte cholesterisch-flüssigkristalline Zusammensetzungen definiert gemäß einem der Ansprüche 4 bis 6.

12. Pigmente nach Anspruch 11, **dadurch gekennzeichnet, daß** es sich um Mehrschichtpigmente handelt.

13. Kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische Zubereitungen bekannten, im UV-Bereich absorbierenden Verbindungen, als photostabile UV-Filter wirksame Mengen von cholesterisch-flüssigkristallinen Zusammensetzungen, definiert gemäß einem der Ansprüche 4 bis 6, enthalten.

14. Kosmetische und pharmazeutische Zubereitungen nach Anspruch 13, enthaltend als UV-Filter cholesterisch-flüssigkristalline Zusammensetzungen in Form von Pigmenten.

15. Kosmetische und pharmazeutische Zubereitungen nach einem der Ansprüche 13 oder 14, enthaltend als UV-Filter cholesterisch-flüssigkristalline Zusammensetzungen in Form von mehrschichtigen Pigmenten.

## Claims

1. A chiral dopant of the formula I
Z¹-Y¹-(A¹)ₘ-Y²-M-Y³-X-Z² I
in which the variables have, independently of one another, the following meanings:
A¹ a spacer selected from the group consisting of
-(CH₂)_{q}-, -(CH₂CH₂O)ᵣCH₂CH₂-, -CH₂CH₂SCH₂CH₂-, -CH₂CH₂NHCH₂CH₂-, 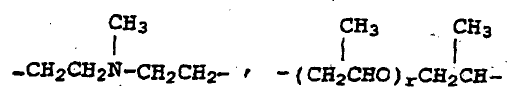 and 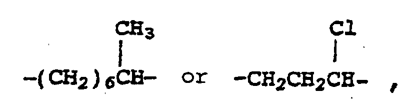 where r is 1 to 3 and q is 1 to 12,
Y¹ to Y³ a chemical bond, -O-, -S-, -C(=O)-, -C(=O)-O-, -O-C(=O)-, -CH=CH-C(=O)-O-, -O-C(=O)-O-, -C(=O)-N(R)- or -(R)N-C(=O)-, -CH₂-O-, -O-CH₂-, -CH=N-, -N=CH- or -N=N-;
M a mesogenic group selected from the group consisting of 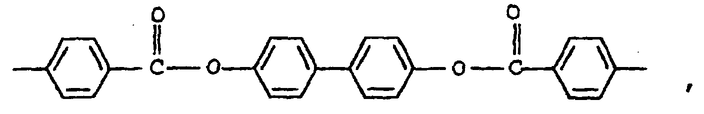 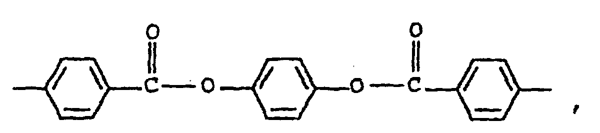 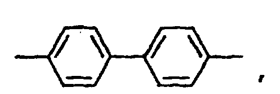 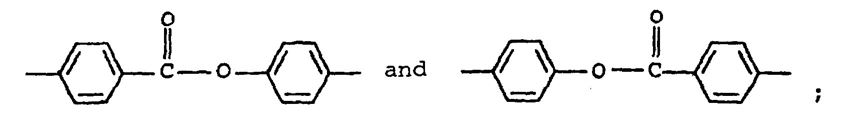
R hydrogen, C₁-C₄-alkyl;
Z¹ and Z² hydrogen, C₁-C₄-alkyl, a polymerizable group or a radical having a polymerizable group, selected from CH₂=CH- , CH≡C- , 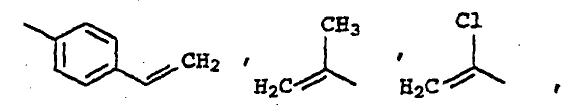 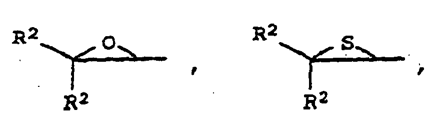 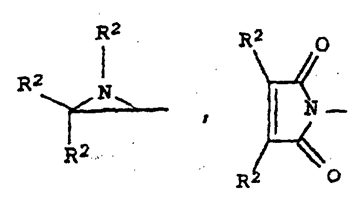 -N=C=O, -N=C=S, -O-C≡N, -COOH, -OH and NH₂,
where the R² radicals may be identical or different and are hydrogen or C₁-C₄-alkyl such as methyl, ethyl, n-propyl, isopropyl, n-butyl, isobutyl, sec-butyl or tert-butyl;
X a dianhydrohexitol residue selected from the group consisting of dianhydrosorbitol, dianhydromannitol and dianhydroiditol;
m 0 or 1,
where the radicals Z¹, Z², Y¹ to Y³ can be identical or different, and at least one Z¹ or Z² radical is a polymerizable group or a radical comprising a polymerizable group.

2. A chiral dopant as claimed in claim 1, in which the variables have, independently of one another, the following meanings:
A¹ a spacer selected from the group consisting of ethylene, propylene, n-butylene, n-pentylene and n-hexylene;
Y¹ to Y³ a chemical bond, -O-, -S-, -C(=O)-, -C(=O)-O-, -O-C(=O)-;
M a mesogenic radical from the group consisting of: 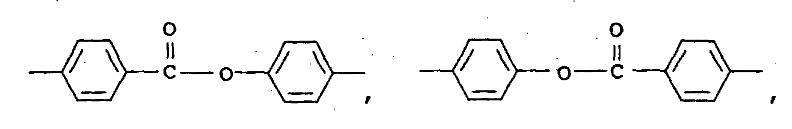 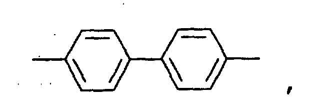 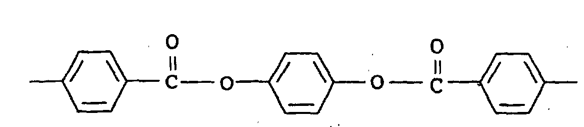 and 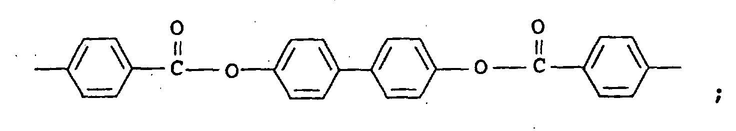
Z¹ and Z² CH₂=CH-, 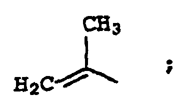
X a dianhydromannitol residue;
m 0 or 1.

3. A chiral dopant as claimed in claim 1 of the formula Ia, 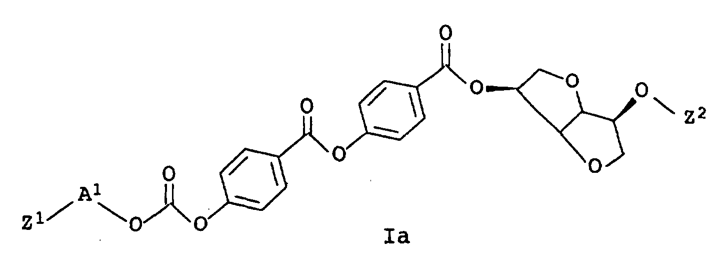 in which the variables have, independently of one another, the following meanings:
A1 a spacer selected from the group consisting of ethylene, propylene, n-butylene, n-pentylene and n-hexylene;
Z¹ and Z² CH₂=CH-,

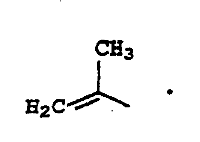

4. A cholesteric liquid crystal composition comprising
a) at least one chiral liquid crystal polymerizable monomer of the formula I
Z¹-Y¹-(A¹)ₘ-Y²-M-Y³-X-Z² I
defined as in claim 1,
with which it is possible to obtain a cholesteric liquid crystal phase with a pitch of less than 450 nm, in which the variables have the meaning stated in claim 1,
or
b) a mixture of
b₁) at least one achiral liquid crystal polymerizable monomer of the formula II
Z³-Y⁶-(A³)ₒ-Y⁷-M¹-Y⁸-(A⁴)ₚ-Y⁹-Z⁴ II
in which the variables have, independently of one another, the following meanings:
A³ and A⁴ a spacer selected from the group consisting of
-(CH₂)_{q}-, -(CH₂CH₂O)ᵣCH₂CH₂-, -CH₂CH₂SCH₂CH₂-, -CH₂CH₂NHCH₂CH₂-, 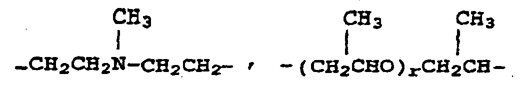 and 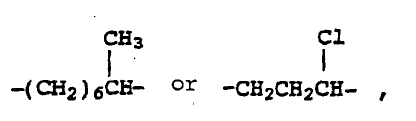 where r is 1 to 3 and q is 1 to 12,
M¹ a mesogenic group selected form the group consisting of 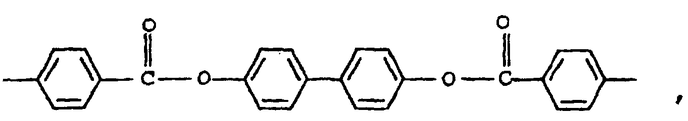 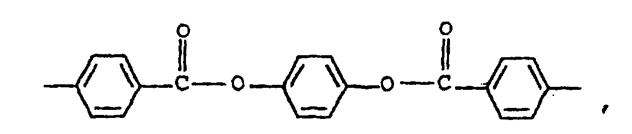 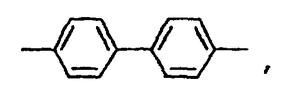 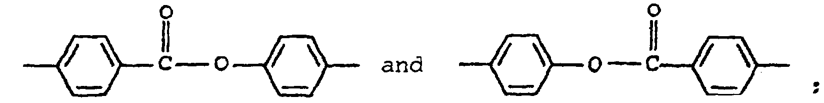
Y⁶ to Y⁹ a chemical bond, -O-, -S-, -C(=O)-, -C(=O)-O-, -O-C(=O)-, -CH=CH-C(=O)-O-, -O-C(=O)-O-, -C(=O)-N(R¹)- or -(R¹)N-C(=O)-, -CH₂-O-, -O-CH₂-, -CH=N-, -N=CH- or -N=N-;
R¹ hydrogen, C₁-C₄-alkyl;
o 0 or 1;
p 0 or 1;
Z³ and Z⁴ hydrogen, C₁-C₄-alkyl, a polymerizable group or a radical having a polymerizable group, selected from CH₂=CH- , CH≡C- , 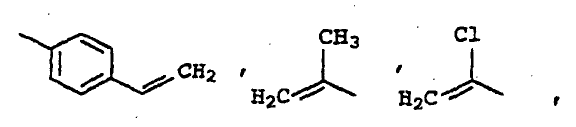 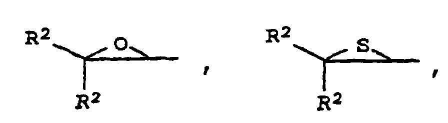 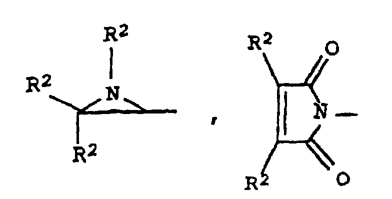 -N=C=O, -N=C=S, -O-C≡N, -COOH, -OH and NH₂,
where the R² radicals may be identical or different and are hydrogen or C₁-C₄-alkyl such as methyl, ethyl, n-propyl, isopropyl, n-butyl, isobutyl, sec-butyl or tert-butyl and where at least one of the variables Z³ or Z⁴ is a polymerizable group or a radical having a polymerizable group, and
b₂) at least one chiral liquid crystal polymerizable monomer of the formula I, defined as in claim 1, with which a cholesteric liquid crystal phase with a pitch of less than 450 nm can be obtained.

5. A cholesteric liquid crystal composition as claimed in claim 4, comprising as chiral liquid crystal polymerizable monomer a) or b₂) at least one chiral compound of the formula Ia, 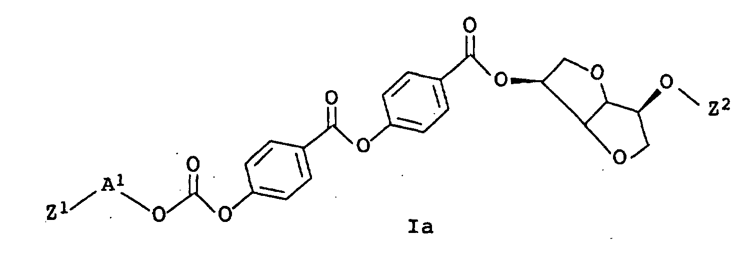 in which the variables have, independently of one another, the following meanings:
A¹ a spacer selected from the group consisting of ethylene, propylene, n-butylene, n-pentylene and n-hexylene;
Z¹ and Z² CH₂=CH-,

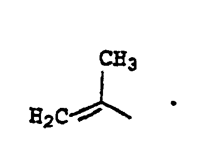

6. A cholesteric liquid crystal composition as claimed in either of claims 4 or 5, which reflects left-circularly polarized light in the UV region.

7. The use of cholesteric liquid crystal compositions as defined in any of claims 4 to 6 as UV filters in cosmetic and pharmaceutical preparations for protecting the human skin or human hair from the sun's rays, alone or together with compounds which absorb in the UV region and are known per se for cosmetic and pharmaceutical preparations.

8. The use of cholesteric liquid crystal compositions as claimed in claim 7 as photostable UV reflectors.

9. The use of cholesteric liquid crystal compositions as defined in any of claims 4 to 6 as UV stabilizers in cosmetic and pharmaceutical formulations.

10. The use of cholesteric liquid crystal compositions as claimed in any of claims 7 to 9 in the form of pigments.

11. A pigment comprising a polymerized cholesteric liquid crystal composition as defined in any of claims 4 to 6.

12. A pigment as claimed in claim 11, which is a multilayer pigment.

13. A cosmetic or pharmaceutical preparation for protecting the human epidermis or human hair from UV light in the range from 280 to 400 nm, which comprises in a cosmetically or pharmaceutically suitable vehicle, alone or together with compounds which absorb in the UV region and are known per se for cosmetic or pharmaceutical preparations, a cholesteric liquid crystal composition as defined in any of claims 4 to 6 in amounts effective as photostable UV filters.

14. A cosmetic or pharmaceutical preparation as claimed in claim 13, comprising a cholesteric liquid crystal composition in the form of a pigment as UV filter.

15. A cosmetic or pharmaceutical preparation as claimed in either of claims 13 or 14, comprising a cholesteric liquid crystal composition in the form of a multilayer pigment as UV filter.

## Revendications

1. Dopants chiraux de formule générale I,
Z¹-Y¹-(A¹)ₘ-Y²-M-Y³-X-Z² I
dans laquelle les variables ont, indépendamment l'une de l'autre, la signification suivante:
A¹ un espaceur, choisi dans le groupe consistant en
-(CH₂)_{q}-, -(CH₂CH₂O)ᵣCH₂CH₂-, -CH₂CH₂SCH₂CH₂-, -CH₂CH₂NHCH₂CH₂-, 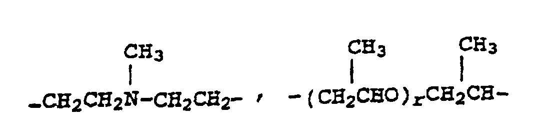 et 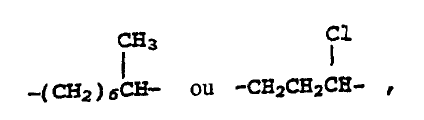 où r vaut 1 à 3 et q vaut 1 à 12;
Y¹ à Y³ une liaison chimique, -O-, -S-, -C(=O)-, -C(=O)-O-, -O-C(=O)-, -CH=CH-C(=O)-O-, -O-C(=O)-O-, -C(=O)-N(R)- ou -(R)N-C(=O)-, -CH₂-O-, -O-CH₂-, -CH=N-, -N=CH- ou -N=N-;
M un groupe mésogène, choisi dans le groupe consistant en 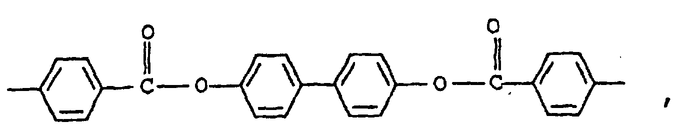 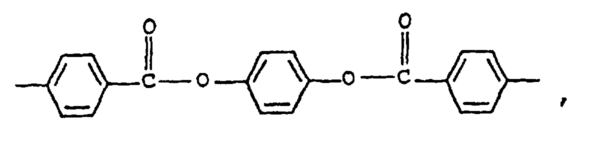 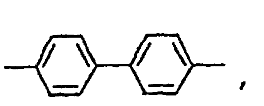 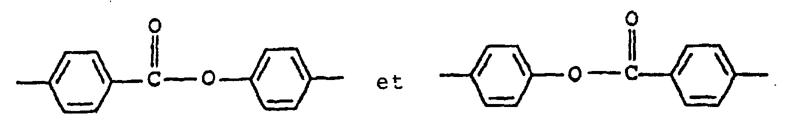
R hydrogène, alkyle en C₁-C₄;
Z¹ et Z² hydrogène, alkyle en C₁-C₄, un groupe polymérisable ou un reste qui porte un groupe polymérisable, choisi parmi:
CH₂=CH-, CH≡C-, 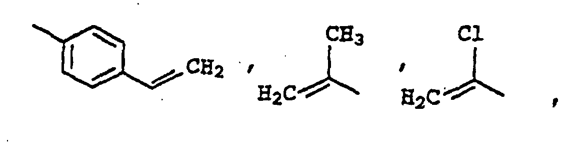 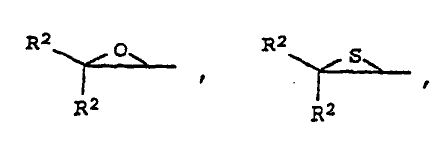 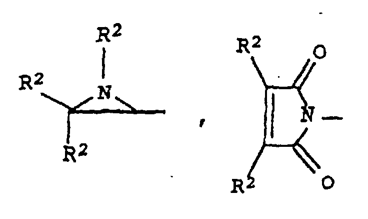 -N=C=O, -N=C=S, -O-C≡N, -COOH, -OH et NH₂,
tandis que les restes R² peuvent être identiques ou différents et représentent l'hydrogène ou un groupe alkyle en C₁-C₄, tel que méthyle, éthyle, n-propyle, iso-propyle, n-butyle, iso-butyle, sec-butyle ou tert-butyle;
X un reste dianhydrohexitol, choisi dans le groupe consistant en dianhydrosorbitol, dianhydromannitol et dianhydroiditol;
m 0 ou 1,
tandis que les restes Z¹, Z², Y¹ à Y³ peuvent être identiques ou différents et au moins un reste Z¹ ou Z² désigne un groupe polymérisable ou un reste qui contient un groupe polymérisable.

2. Dopants chiraux selon la revendication 1, dans lesquels les variables ont indépendamment l'une de l'autre la signification suivante:
A¹ un espaceur, choisi dans le groupe consistant en l'éthylène, le propylène, le n-butylène, le n-pentylène et le n-hexylène;
Y¹ à Y³ une liaison chimique, -O-, -S-, -C(=O)-, -C(=O)-O-, -O-C(=O)-;
M un reste mésogène, choisi dans le groupe consistant en 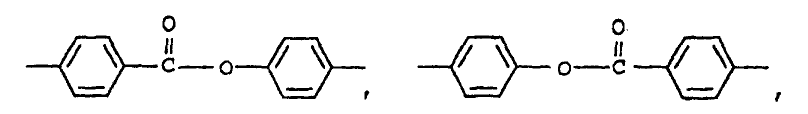 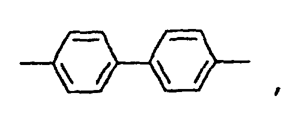 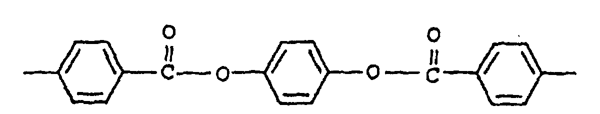 et 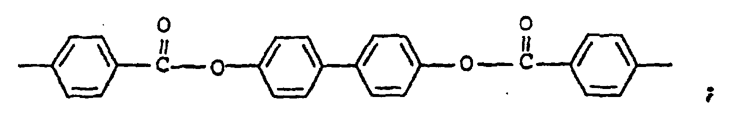
Z¹ et Z² CH₂=CH-, 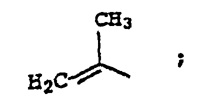
X un reste dianhydromannitol;
m 0 ou 1.

3. Dopants chiraux selon la revendication 1 de formule générale la, 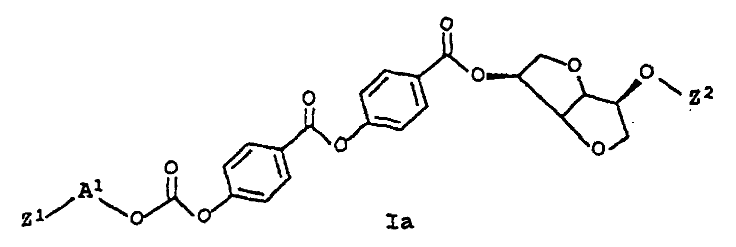 dans laquelle les variables ont indépendamment l'une de l'autre la signification suivante:
A¹ un espaceur, choisi dans le groupe consistant en l'éthylène, le propylène, le n-butylène, le n-pentylène et le n-hexylène;
Z¹ et Z² CH₂=CH-,

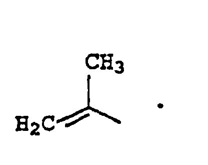

4. Compositions de cristaux liquides cholestériques, contenant
a) au moins un monomère polymérisable, en cristaux liquides chiraux, de formule générale I,
Z¹-Y¹-(A¹)ₘ-Y²-M-Y³-X-Z² I
définie selon la revendication 1,
avec lequel on peut obtenir une phase de cristaux liquides cholestériques ayant un pas inférieur à 450 nm, tandis que les variables ont la signification indiquée dans la revendication 1
ou
b) un mélange
b₁) d'au moins un monomère polymérisable en cristaux liquides achiraux de formule générale II
Z³-Y⁶-(A³)ₒ-Y⁷-M¹-Y⁸-(A⁴)ₚ-Y⁹-Z⁴ II
où les variables ont, indépendamment l'une de l'autre la signification suivante:
A³ et A⁴ un espaceur, choisi dans le groupe consistant en
-(CH₂)_{q}-, -(CH₂CH₂O)ᵣCH₂CH₂-, -CH₂CH₂SCH₂CH₂-, -CH₂CH₂NHCH₂CH₂-, 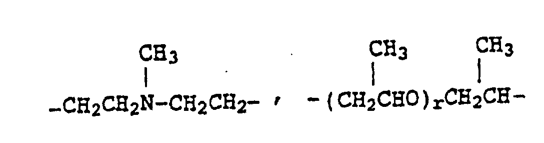 et 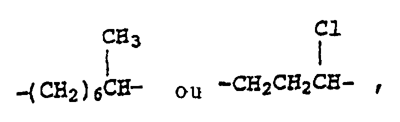 où r vaut 1 à 3 et q vaut 1 à 12;
M¹ un groupe mésogène, choisi dans le groupe consistant en 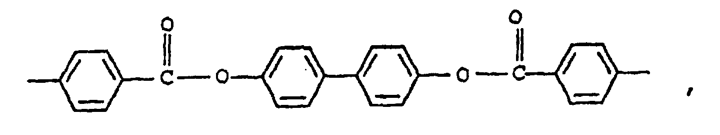 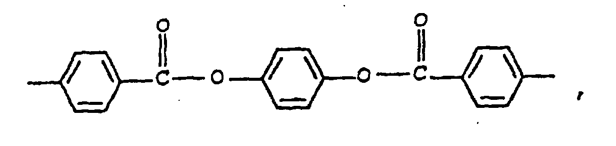 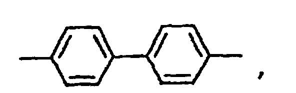 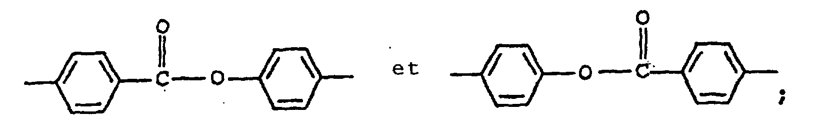
Y⁶ à Y⁹ une liaison chimique, -O-, -S-, -C(=O)-, -C(=O)-O-, -O-C(=O)-, -CH=CH-C(=O)-O-, -O-C(=O)-O-, -C(=O)-N(R¹)- ou -(R¹)N-C(=O)-, -CH₂-O-, -O-CH₂-, -CH=N-, -N=CH- ou -N=N-;
R¹ hydrogène, alkyle en C₁-C₄;
o 0 ou 1;
p 0 ou 1;
Z³ et Z⁴ hydrogène, alkyle en C₁-C₄, un groupe polymérisable ou un reste qui porte un groupe polymérisable, choisi parmi
CH₂=CH-, CH≡C-, 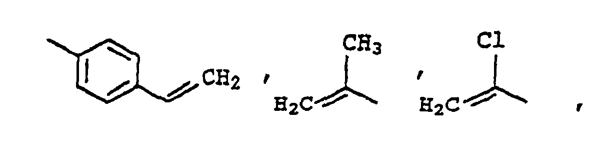 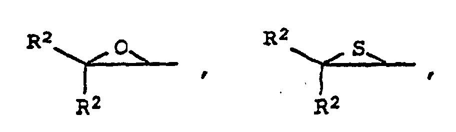 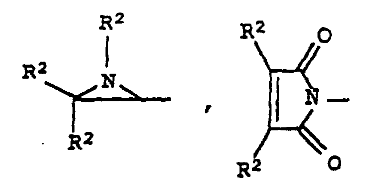 -N=C=O, -N=C=S, -O-C≡N, -COOH, -OH et NH₂,
tandis que les restes R² peuvent être identiques ou différents et désignent l'hydrogène ou un groupe alkyle en C₁-C₄, tel que méthyle, éthyle, n-propyle, iso-propyle, n-butyle, iso-butyle, sec-butyle ou tert-butyle, et tandis qu'au moins un reste Z³ ou Z⁴ représente un groupe polymérisable ou un reste qui contient un groupe polymérisable, et
b₂) d'au moins un monomère polymérisable en cristaux liquides chiraux, de formule I, définie conformément à la revendication 1, avec lequel on peut obtenir une phase de cristaux liquides cholestériques ayant un pas inférieur à 450 nm.

5. Compositions de cristaux liquides cholestériques selon la revendication 4, contenant comme monomère polymérisable, en cristaux liquides chiraux a) ou b₂) au moins un composé chiral de formule générale Ia, 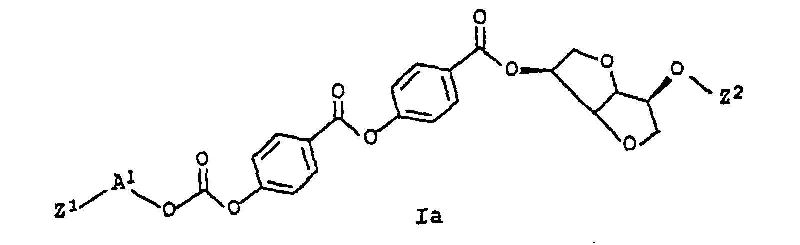 dans laquelle les variables ont indépendamment l'une de l'autre la signification suivante:
A¹ un espaceur, choisi dans le groupe consistant en l'éthylène, le propylène, le n-butylène, le n-pentylène et le n-hexylène;
Z¹ et Z² CH₂=CH-,

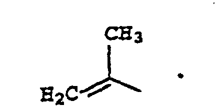

6. Compositions de cristaux liquides cholestériques selon l'une des revendications 4 ou 5, **caractérisées par le fait qu'**elles reflètent la lumière à polarisation circulaire dans le sens de rotation gauche, dans le domaine des UV.

7. Utilisation de compositions de cristaux liquides cholestériques, définies selon l'une des revendications 4 à 6, comme filtre UV dans des préparations cosmétiques et pharmaceutiques pour la protection de la peau humaine ou des cheveux humains contre les rayons solaires, seules ou en combinaison avec des composés connus absorbant dans le domaine des UV, connus en soi pour des préparations cosmétiques et pharmaceutiques.

8. Utilisation de compositions de cristaux liquides cholestériques selon la revendication 7 comme réflecteurs d'UV photostables.

9. Utilisation de compositions de cristaux liquides cholestériques, définies selon les revendications 4 à 6, comme stabilisants vis-à-vis des UV dans des formulations cosmétiques et pharmaceutiques.

10. Utilisation de compositions de cristaux liquides cholestériques selon l'une des revendications 7 à 9 sous forme de pigments.

11. Pigments contenant des compositions de cristaux liquides cholestériques définies selon l'une des revendication 4 à 6.

12. Pigments selon la revendication 11, **caractérisé par le fait qu'**il s'agit de pigments multicouches.

13. Préparations cosmétiques et pharmaceutiques pour la protection de l'épiderme humain ou des cheveux humains contre la lumière UV dans le domaine de 280 à 400 nm, **caractérisées par le fait qu'**elles contiennent dans un support approprié du point de vue cosmétique et pharmaceutique, seuls ou conjointement avec des composés absorbant dans le domaine UV, connus en soi pour des préparations cosmétiques et pharmaceutiques, des quantités efficaces à titre de filtre UV photostable de compositions en cristaux liquides cholestériques, définies selon les revendications 4 à 6.

14. Préparations cosmétiques et pharmaceutiques selon la revendication 13, contenant à titre de filtre UV des compositions en cristaux liquides cholestériques sous forme de pigments.

15. Préparations cosmétiques et pharmaceutiques selon l'une des revendications 13 ou 14, contenant à titre de filtre UV des compositions en cristaux liquides sous forme de pigments multicouches.
